# EUROPEAN PATENT APPLICATION

(11) **EP 4 502 170 A1**
(43) Date of publication of application: **05.02.2025**
(21) Application number: 23779505.9
(22) Date of filing: 13.03.2023
(51) Int. Cl.: C12Q 1/02, A61K 35/28, A61L 27/38, C12N 5/077, C12N 5/0775, G01N 21/64, G01N 33/58, A61P 19/00, A61P 21/00, A61P 37/06

(54) **QUALITY EVALUATION METHOD OF MESENCHYMAL STEM CELLS**

(30) Priority: 31.03.2022 JP 2022059920; 09.12.2022 JP 2022196791
(71) Applicant: Cyto-Facto Inc., Kobe-shi, Hyogo 650-0047 (JP)
(72) Inventor: KAWAMATA, Shin, Kobe-shi, Hyogo 650-0047 (JP); YAMAMOTO, Takako, Kobe-shi, Hyogo 651-0097 (JP)
(74) Representative: J A Kemp LLP
(86) International application number: PCT/JP2023/009633
(87) International publication number: WO 2023/189485

(57) **Abstract**

The present invention provides a method for evaluating the quality of mesenchymal stem cells, said method including a step for evaluating the cells on the basis of the measured consumption amounts of pyruvic acid, cystine and serine in the cells. The present invention also provides a method for evaluating the quality of mesenchymal stem cells, said method including a step for evaluating the cells on the basis of the measured mitochondrial size in the cells.

## Description

### TECHNICAL FIELD

The present invention relates to a method for evaluating quality of mesenchymal stem cells using the mitochondrial size as an indicator. The present invention also relates to a method for producing mesenchymal stem cells evaluated for the quality using this evaluation method, a method for producing mesenchymal cells using the mesenchymal stem cells produced using this production method, and the like. Furthermore, the present invention also relates to a method for evaluating a medium for mesenchymal stem cells using the mitochondrial size as an indicator.

The present invention also relates to a method for evaluating quality of mesenchymal stem cells using the consumptions of pyruvic acid, cystine, and serine in the culture medium as indicators. In addition, the present invention also relates to a method for producing mesenchymal stem cells evaluated for the quality using this evaluation method, a method for producing mesenchymal cells using the mesenchymal stem cells produced using this production method, and the like. Furthermore, the present invention also relates to a method for evaluating a medium for mesenchymal stem cells using the consumptions of pyruvic acid, cystine, and serine in the culture medium as indicators.

### BACKGROUND ART

Mesenchymal stem cells (MSCs), which are one type of somatic stem cells, have few ethical issues related to cell collection and are capable of differentiating into bones, cartilages, fats, and the like, and therefore, basic research, clinical applications, and the like have been actively conducted using the mesenchymal stem cells. When the mesenchymal stem cells are used for medical purposes and research purposes, quality control on the mesenchymal stem cells is very important. For example, in the case of medical applications, the cell quality greatly affects not only patients' safety but also the reliability and stability of therapeutic effects. In the case of research as well, the cell quality has a great impact on the success and reproducibility of experiments.

However, for example, when mesenchymal stem cells are used for transplantation, it is necessary to conduct expansion culture of the mesenchymal stem cells and repeat passaging in order to prepare the required number of cells for the cell transplantation. Because the mesenchymal stem cells constitute a non-homogeneous population, the stem cells constitute a heterogeneous cell population, and their quality (differentiation potential, proliferative capacity, undifferentiation properties, etc.) greatly varies depending on the culture conditions, the cell passage number, and the like.

Accordingly, in recent years, it is required to establish a technology to evaluate the quality of mesenchymal stem cells. For example, a method in which the existence ratio of cells expressing Ror2 or Fzd5 in a cultured cell population is used as an indicator (Patent Literature 1) and other methods have been reported as methods for evaluating quality of mesenchymal stem cells, but are still not entirely satisfactory. Therefore, there is demand for a more reliable, simpler, and quicker method for evaluating quality of mesenchymal stem cells.

### Citation List

### Patent Literature

Patent Literature 1: WO 2016/017795

### SUMMARY OF INVENTION

### Technical Problem

Accordingly, it is an object of the present invention to provide a more reliable, simpler, and quicker method for evaluating quality of mesenchymal stem cells. Also, it is another object of the present invention to provide a method for producing mesenchymal stem cells evaluated for the quality using this evaluation method, a method for producing mesenchymal cells using the mesenchymal stem cells produced using this production method, and the like. Furthermore, it is yet another object of the present invention to provide a method for evaluating a medium for mesenchymal stem cells using the mitochondrial size as an indicator. In addition, it is yet another object of the present invention to provide a method for evaluating a medium for mesenchymal stem cells using the consumptions of pyruvic acid, cystine, and serine in the culture medium as indicators.

### Solution to Problem

First, the inventors of the present invention focused on oxidative stress, which is considered to be one of the factors that deteriorate the quality and the like of mesenchymal stem cells during expansion culture of the stem cells. In particular, the inventors focused on the production of reactive oxygen species, which is one type of oxidative stress, and conducted research on novel indicators for evaluation of the quality of mesenchymal stem cells. As a result, the inventors focused on the presence of mitochondria, which is one of the causes of the production of reactive oxygen species, and came up with an idea that there is some kind of relationship between the culture period of mesenchymal stem cells and the mitochondria in these cells.

As a result of extensive research conducted based on the idea above, the inventors surprisingly confirmed that, as mesenchymal stem cells were repeatedly passaged, mitochondria in the cell fused to each other and increased in size, which led to fatigue (deterioration), and found that there was relationship between the mitochondrial sizes in mesenchymal stem cells and the passage number. As a result of further research on this result, the inventors found that the quality of mesenchymal stem cells could be evaluated based on the mitochondrial size or a change in mitochondrial size. Also, the inventors confirmed that mitochondrial fatigue varied depending on the types of media in which mesenchymal stem cells were cultured. As a result of further research on this result as well, the inventors also found that a medium suitable for culture of mesenchymal stem cells could be screened based on the mitochondrial size or a change in mitochondrial size. As a result of further research based on these findings, the inventors have achieved the present invention in which the mitochondrial size is used as an indicator.

Incidentally, the inventors focused on removal of reactive oxygen species in a living organism in addition to the production of the species and the like focused on in the description above, and came up with an idea that there is some kind of relationship between the culture period of mesenchymal stem cells and pathways (e.g., TCA cycle) and substances (e.g., antioxidative substances (such as glutathione)) involved in the production and removal of reactive oxygen species. As a result of extensive research conducted based on the idea above, the inventors surprisingly found that, as mesenchymal stem cells were repeatedly passaged, pyruvic acid, cystine, and serine were excessively taken up by the cells. Since pyruvic acid is a substance involved in the TCA cycle and cystine and serine are substances involved in glutathione synthesis in a living organism, the inventors confirmed that the idea above was correct.

Accordingly, the inventors conducted further research on this result. As a result, the inventors found that the quality of mesenchymal stem cells could be evaluated based on changes in amounts of pyruvic acid, cystine, and serine in the culture medium of the cells. Also, the inventors confirmed that changes in amounts of pyruvic acid, cystine, and serine in the culture medium of the mesenchymal stem cells varied depending on the types of media in which the cells were cultured. As a result of further research on this result as well, the inventors also found that a medium suitable for culture of mesenchymal stem cells could be screened based on the changes in amounts of pyruvic acid, cystine, and serine. As a result of further research based on these findings, the inventors have achieved the present invention in which the consumptions of pyruvic acid, cystine, and serine in the culture medium are used as indicators.

That is to say, the present invention is as follows.
[1] A method for evaluating quality of mesenchymal stem cells, including a step of evaluating the cells based on
   (i) measurements of consumptions of pyruvic acid, cystine, and serine by the cells, or
   (ii) measurements of mitochondrial sizes in the cells.
[2] The method according to [1], wherein the consumptions of pyruvic acid, cystine, and serine are calculated based on differences in abundances of pyruvic acid, cystine, and serine in a medium between before and after the start of culture of the cells.
[3] The method according to [2], wherein the abundances are concentrations.
[4] The method according to any one of [1] to [3], wherein the step of evaluating the cells is conducted based on differences in the consumptions of pyruvic acid, cystine, and serine between before and after a passage.
[5] The method according to [4], further including a step of selecting cells in which the differences in the consumptions of pyruvic acid, cystine, and serine between before and after a passage are greater than or equal to predetermined values.
[6] The method according to [4], further including a step of selecting cells in which the differences in the consumptions of pyruvic acid, cystine, and serine between before and after a passage are smaller than or equal to predetermined values.
[7] The method according to [1], wherein the mitochondrial sizes are calculated using fluorescence intensities of fluorescence-labeled mitochondria.
[8] The method according to [7], wherein the fluorescence intensities of the mitochondria reflect a mitochondrial charge.
[9] The method according to any one of [1], [7], and [8], further including a step of selecting cells with a CV value smaller than or equal to a predetermined value.
[10] A method for producing mesenchymal stem cells evaluated for quality, including a step of evaluating the cells based on
   (i) measurements of consumptions of pyruvic acid, cystine, and serine by the cells, or
   (ii) measurements of mitochondrial sizes in the cells.
[11] Mesenchymal stem cells obtained using the method according to [10].
[12] A method for producing mesenchymal cells, including a step of inducing differentiation of the mesenchymal stem cells according to [11].
[13] Mesenchymal cells obtained using the method according to [12].
[14] A cell transplantation therapy agent including the mesenchymal stem cells according to [11] or the mesenchymal cells according to [13].
[15] A method for evaluating a medium for mesenchymal stem cells, including a step of evaluating the medium based on
   (i) measurements of consumptions of pyruvic acid, cystine, and serine by the cells, or
   (ii) measurements of mitochondrial sizes in the cells.
[16] The method according to [15], wherein the consumptions of pyruvic acid, cystine, and serine are calculated based on differences in abundances of pyruvic acid, cystine, and serine in a medium between before and after the start of culture of the cells.
[17] The method according to [15], wherein the mitochondrial sizes are calculated using fluorescence intensities of fluorescence-labeled mitochondria.
[18] A method for treating an injury of a tissue or a disease, including administering or transplanting an effective amount of the mesenchymal stem cells according to [11] or the mesenchymal cells according to [13].
[19] The mesenchymal stem cells according to [11] or the mesenchymal cells according to [13] for use to treat an injury of a tissue or a disease.
[20] Use of the mesenchymal stem cells according to [11] or the mesenchymal cells according to [13] to produce a therapeutic agent for an injury of a tissue or a disease.

### Advantageous Effects of Invention

With the present invention, it is possible to more reliably, more simply, and more quickly evaluate the quality of mesenchymal stem cells. Using this evaluation method makes it possible to produce mesenchymal stem cells with stable quality (differentiation potential, proliferative capacity, undifferentiation properties, etc.). Differentiating mesenchymal stem cells with stable quality also makes it possible to produce mesenchymal cells with stable quality more efficiently and reproducibly. Although mesenchymal stem cells and mesenchymal cells are important to the cell transplantation therapy and are required to have stable quality, the production method of the present invention makes it easy to secure the required number of cells for the cell transplantation treatment. Furthermore, the mesenchymal stem cells and the mesenchymal cells obtained using the production methods of the present invention have stable quality and are reproducibly produced, and are thus particularly suitable for application to the cell transplantation treatment. For example, the mesenchymal stem cells obtained using the production method of the present invention can be suitably used for treatment (cell transplantation therapy) of a disease with an injury to a bone, cartilage, or muscle. In addition, with the present invention, it is also possible to evaluate a medium for mesenchymal stem cells. Using this evaluation method makes it possible to select a medium with which deterioration of the quality of mesenchymal stem cells caused by passaging can be further avoided, thus making it possible to supply mesenchymal stem cells and mesenchymal cells with stable quality more efficiently and reproducibly.

### Brief Description of Drawings

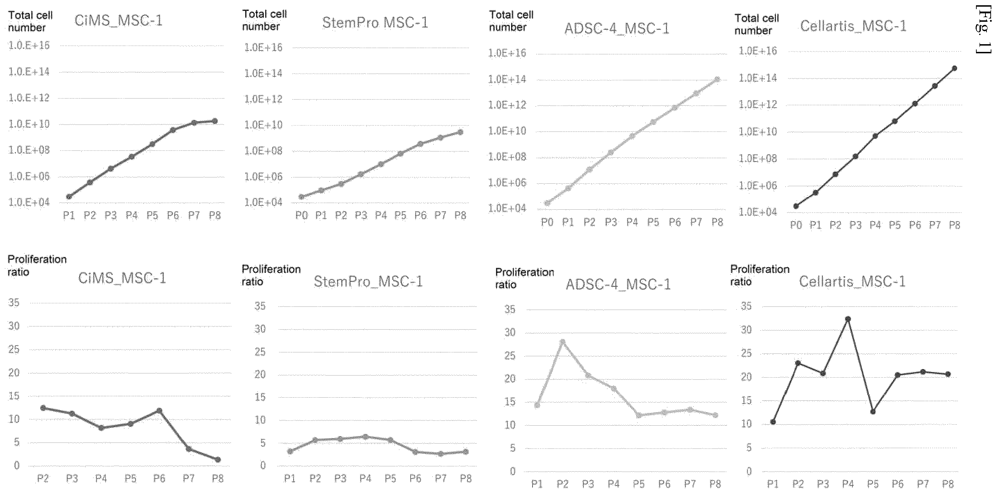

[FIG. 1] FIG. 1 shows proliferation curves (upper diagrams in FIG. 1) and proliferation ratios (lower diagrams in FIG. 1) of mesenchymal stem cells in various media (CiMS, StemPro, ADSC-4, and Cellartis) up to the eighth passage (P8).

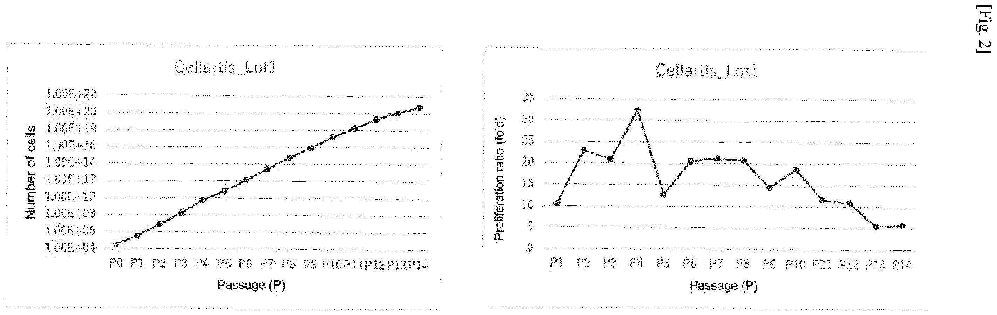

[FIG. 2] FIG. 2 shows a proliferation curve (left diagram in FIG. 2) and a proliferation ratio (right diagram in FIG. 2) of mesenchymal stem cells in Cellartis up to the fourteenth passage (P14).

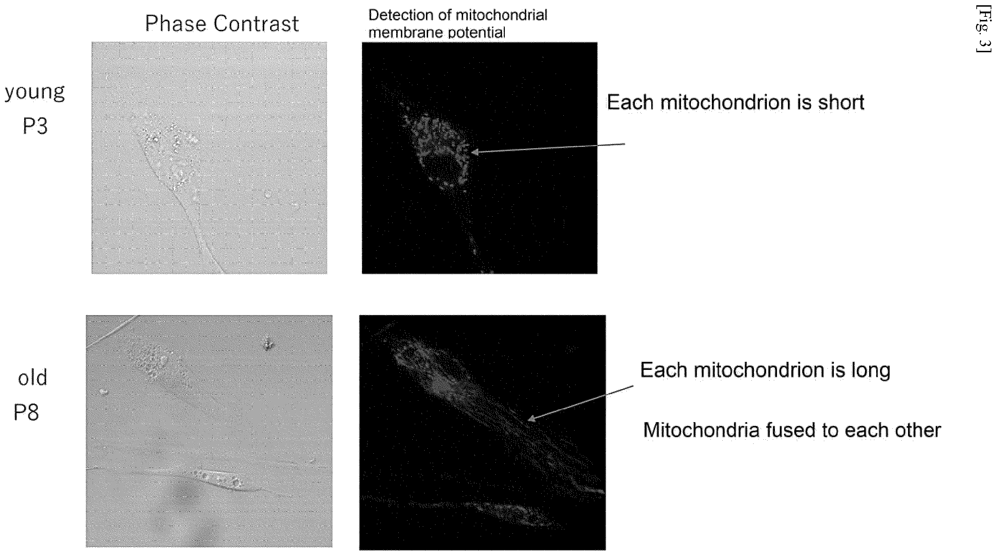

[FIG. 3] FIG. 3 shows diagrams illustrating detection of mitochondrial membrane potential in mesenchymal stem cells (derived from fat). The upper diagrams in FIG. 3 show the mitochondrial state at the third passage (P3), and the lower diagrams in FIG. 3 show the mitochondrial state at the eighth passage (P8).

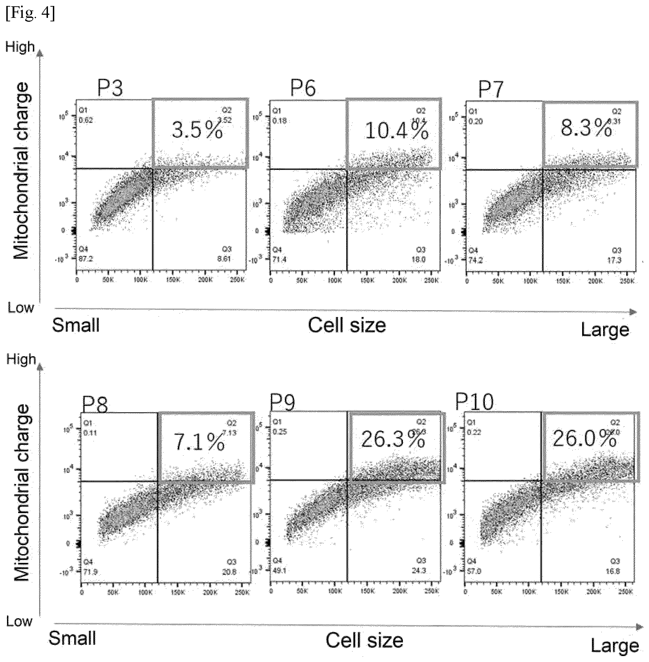

[FIG. 4] FIG. 4 shows diagrams illustrating mitochondrial changes at passages (the third passage (P3), the sixth passage (P6), the seventh passage (P7), the eighth passage (P8), the ninth passage (P9), and the tenth passage (P10)) when mesenchymal stem cells were cultured in CiMS (first trial). The figures (3.5%, 10.4%, 8.3%, 7.1%, 26.3%, and 26.0%) in the upper right portions of the diagrams indicate the percentages of cells with a mitochondrial charge of 5.0 K or more and a cell size of 120 K or more with respect to total cells.

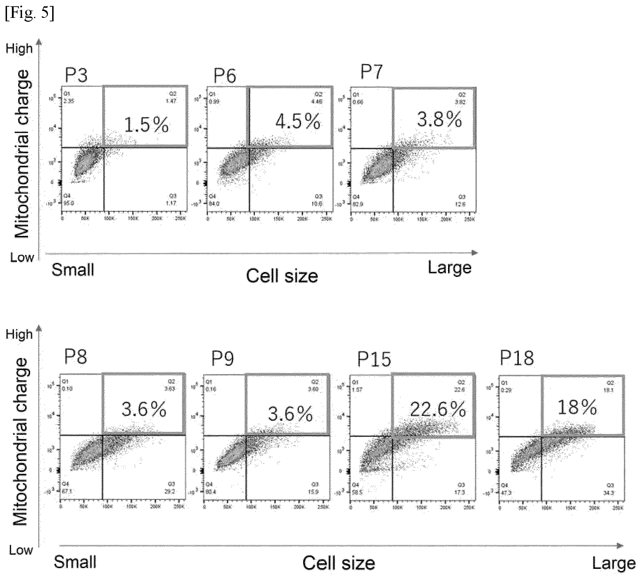

[FIG. 5] FIG. 5 shows diagrams illustrating mitochondrial changes at passages (the third passage (P3), the sixth passage (P6), the seventh passage (P7), the eighth passage (P8), the ninth passage (P9), the fifteenth passage (P15), and the eighteenth passage (P18)) when mesenchymal stem cells were cultured in Cellartis (first trial). The figures (1.5%, 4.5%, 3.8%, 3.6%, 3.6%, 22.6%, and 18%) in the upper right portions of the diagrams indicate the percentages of cells with a mitochondrial charge of 2.5 K or more and a cell size of 90 K or more with respect to total cells.

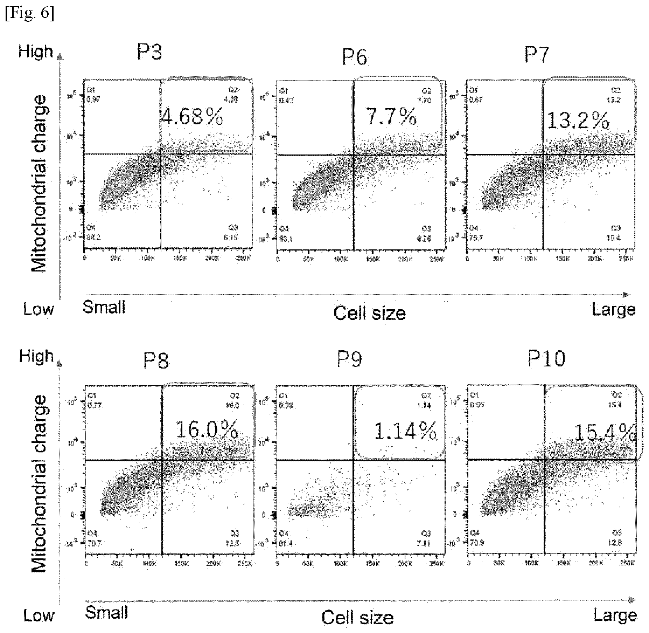

[FIG. 6] FIG. 6 shows diagrams illustrating mitochondrial changes at passages (the third passage (P3), the sixth passage (P6), the seventh passage (P7), the eighth passage (P8), the ninth passage (P9), and the tenth passage (P10)) when mesenchymal stem cells were cultured in CiMS (second trial). The figures (4.68%, 7.7%, 13.2%, 16.0%, 1.14%, and 15.4%) in the upper right portions of the diagrams indicate the percentages of cells with a mitochondrial charge of greater than 5.0 K and a cell size of greater than 120 K with respect to total cells.

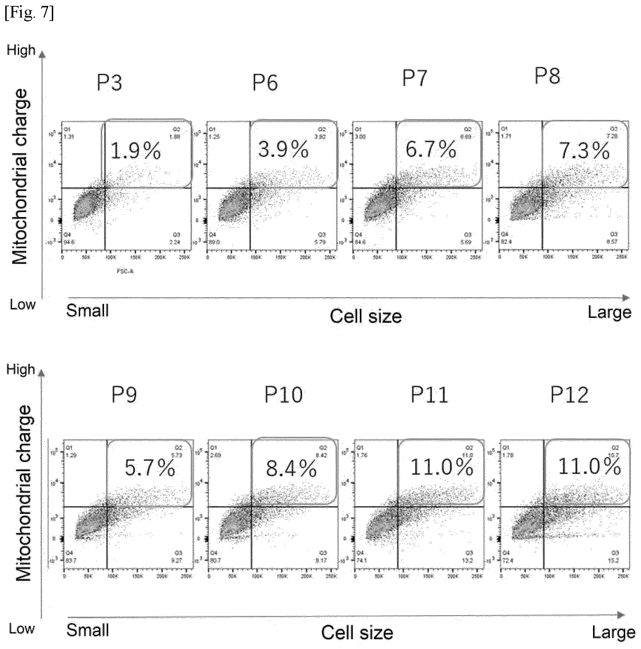

[FIG. 7] FIG. 7 shows diagrams illustrating mitochondrial changes at passages (the third passage (P3), the sixth passage (P6), the seventh passage (P7), the eighth passage (P8), the ninth passage (P9), the tenth passage (P10), the eleventh passage (P11), and the twelfth passage (P12)) when mesenchymal stem cells were cultured in Cellartis (second trial). The figures (1.9%, 3.9%, 6.7%, 7.3%, 5.7%, 8.4%, 11.0%, and 11.0%) in the upper right portions of the diagrams indicate the percentages of cells with a mitochondrial charge of 2.5 K or more and a cell size of 90 K or more with respect to total cells.

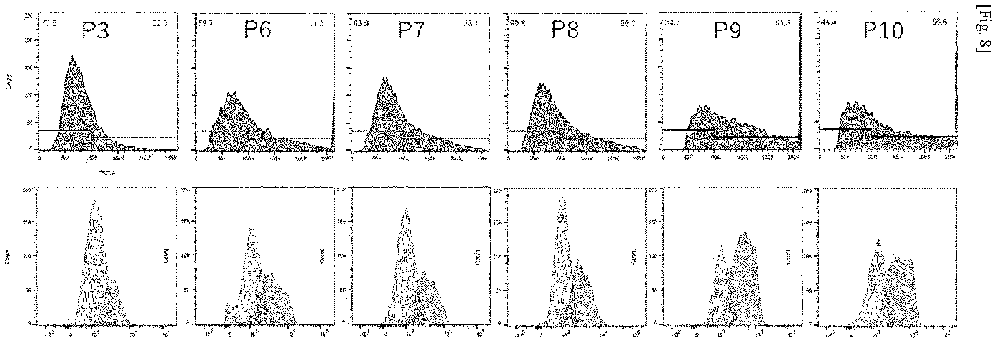

[FIG. 8] FIG. 8 shows diagrams illustrating the analysis results of mitochondrial data at passages (the third passage (P3), the sixth passage (P6), the seventh passage (P7), the eighth passage (P8), the ninth passage (P9), and the tenth passage (P10)) when mesenchymal stem cells were cultured in CiMS/VTN. The figures (77.5 and 22.5 (P3), 58.7 and 41.3 (P6), 63.9 and 36.1 (P7), 60.8 and 39.2 (P8), 34.7 and 65.3 (P9), and 44.4 and 55.6 (P10)) in the upper portions of the upper diagrams in FIG. 8 indicate the percentages of cells contained in groups formed by dividing the cells based on a particular cell size (100 k). The right spike (light gray) in each of the lower diagrams in FIG. 8 indicates the brightness of mitochondria in the cells with a size of 1000 K or less, and the left spike (gray) indicates the brightness of mitochondria in the cells with a size of 1000 K or more. It is found from the lower diagrams in FIG. 8 that the percentage of the brightness of the gray spike (mitochondria in the cells with a size of 1000 K or more) increases as the passaging is repeated.

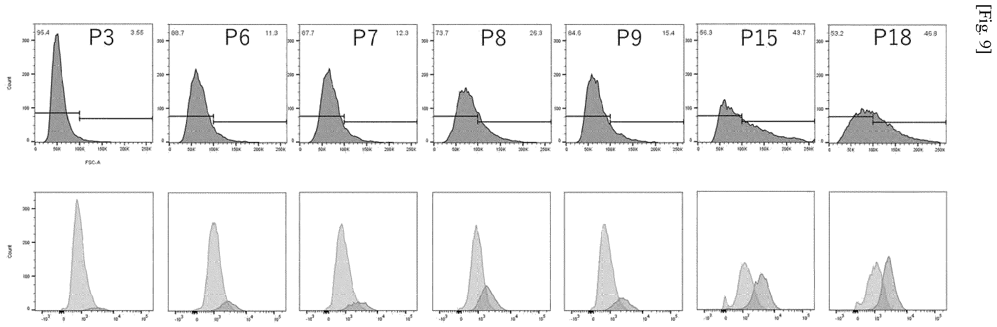

[FIG. 9] FIG. 9 shows diagrams illustrating the analysis results of mitochondrial data at passages (the third passage (P3), the sixth passage (P6), the seventh passage (P7), the eighth passage (P8), the ninth passage (P9), the fifteenth passage (P15), and the eighteenth passage (P18)) when mesenchymal stem cells were cultured in Cellartis. The figures (96.4 and 3.55 (P3), 88.7 and 11.3 (P6), 87.7 and 12.3 (P7), 73.7 and 26.3 (P8), 84.6 and 15.4 (P9), 56.3 and 43.7 (P15), and 53.2 and 46.8 (P18)) in the upper portions of the upper diagrams in FIG. 9 indicate the percentages of cells contained in groups formed by dividing the cells based on a particular cell size (100 k). The right spike (light gray) in each of the lower diagrams in FIG. 9 indicates the brightness of mitochondria in the cells with a size of 1000 K or less, and the left spike (gray) indicates the brightness of mitochondria in the cells with a size of 1000 K or more. It is found from the lower diagrams in FIG. 9 that the percentage of the gray spike (the brightness of mitochondria in the cells with a size of 1000 K or more) increases as the passaging is repeated.

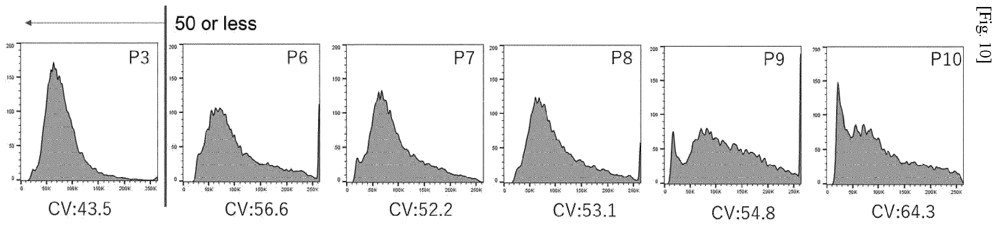

[FIG. 10] FIG. 10 shows diagrams illustrating the analysis results of cell sizes (CV values) at passages (the third passage (P3), the sixth passage (P6), the seventh passage (P7), the eighth passage (P8), the ninth passage (P9), and the tenth passage (P10)) when mesenchymal stem cells were cultured in CiMS/VTN.

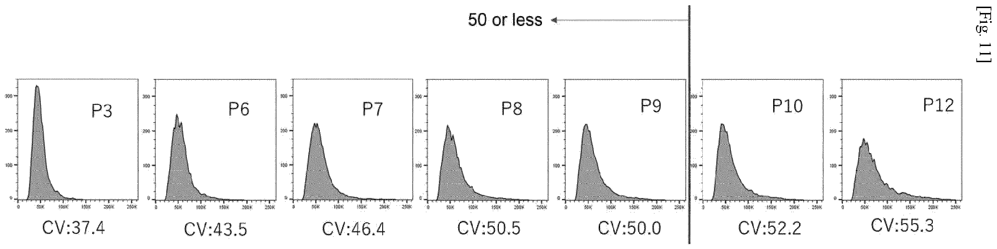

[FIG. 11] FIG. 11 shows diagrams illustrating the analysis results of cell sizes (CV values) at passages (the third passage (P3), the sixth passage (P6), the seventh passage (P7), the eighth passage (P8), the ninth passage (P9), the tenth passage (P10), and the twelfth passage (P12)) when mesenchymal stem cells were cultured in Cellartis.

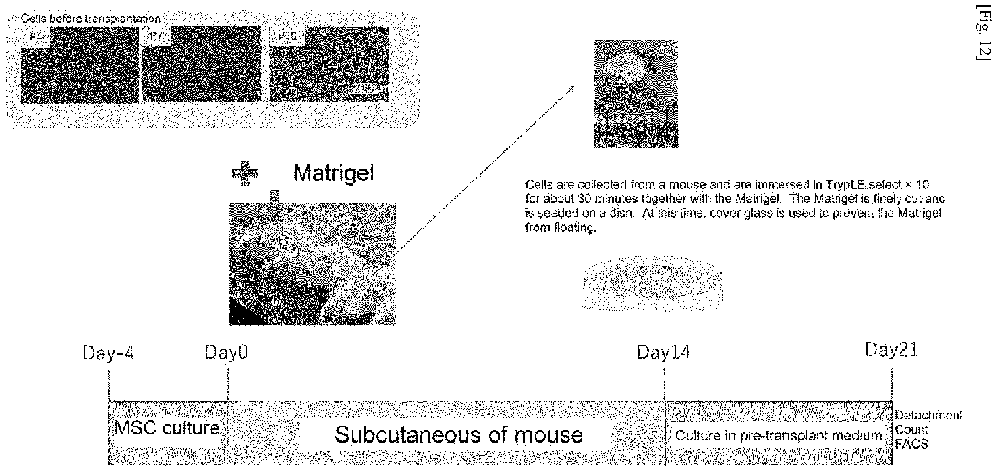

[FIG. 12] FIG. 12 shows diagrams illustrating procedures, from transplantation of mesenchymal stem cells to culture of collected transplanted cells, and the like used in a viability test (Example 5) for the mesenchymal stem cells in an animal performed with the passage number being varied.

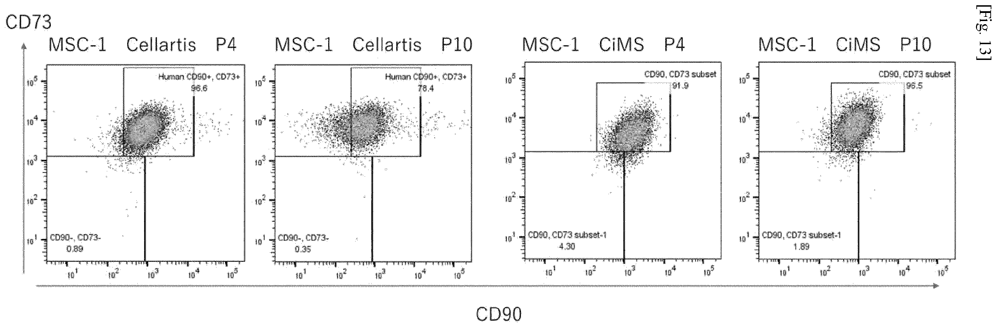

[FIG. 13] FIG. 13 shows diagrams illustrating the results of flow cytometry on mesenchymal stem cells before transplantation into an animal in the viability test (Example 5) for the mesenchymal stem cells in the animal performed with the passage number being varied.

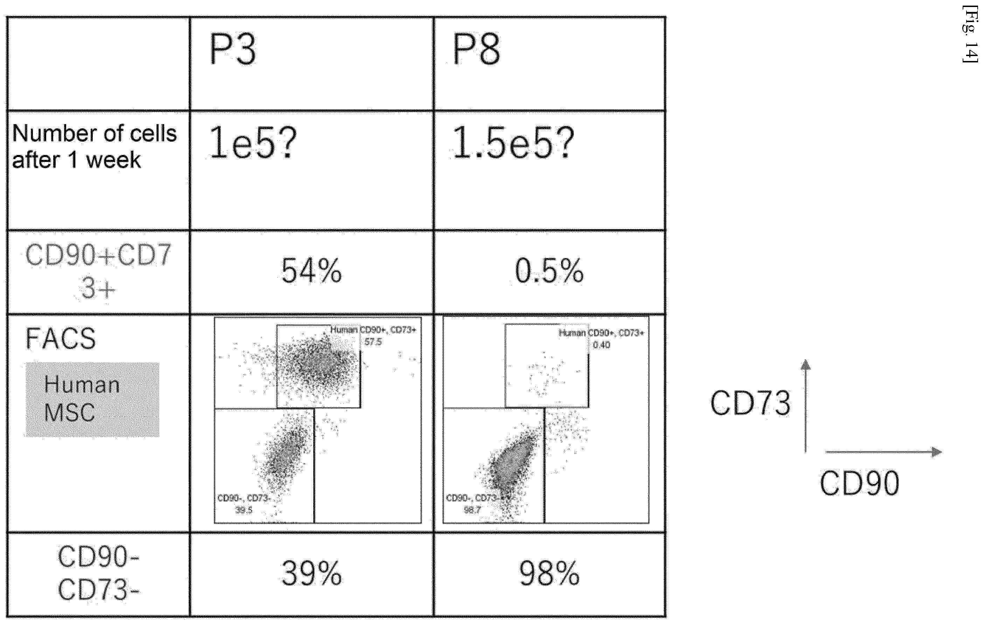

[FIG. 14] FIG. 14 shows a diagram illustrating the results of culture (reculture) of transplanted cells collected after transplantation of mesenchymal stem cells (CiMS (Nipro)) in the viability test (Example 5) for the mesenchymal stem cells in the animal performed with the passage number being varied. As shown in FIG. 14, the proliferated mesenchymal stem cells made up about 54% at the third passage (P3), whereas the proliferated mesenchymal stem cells made up about 0.5% at the eighth passage (P8).

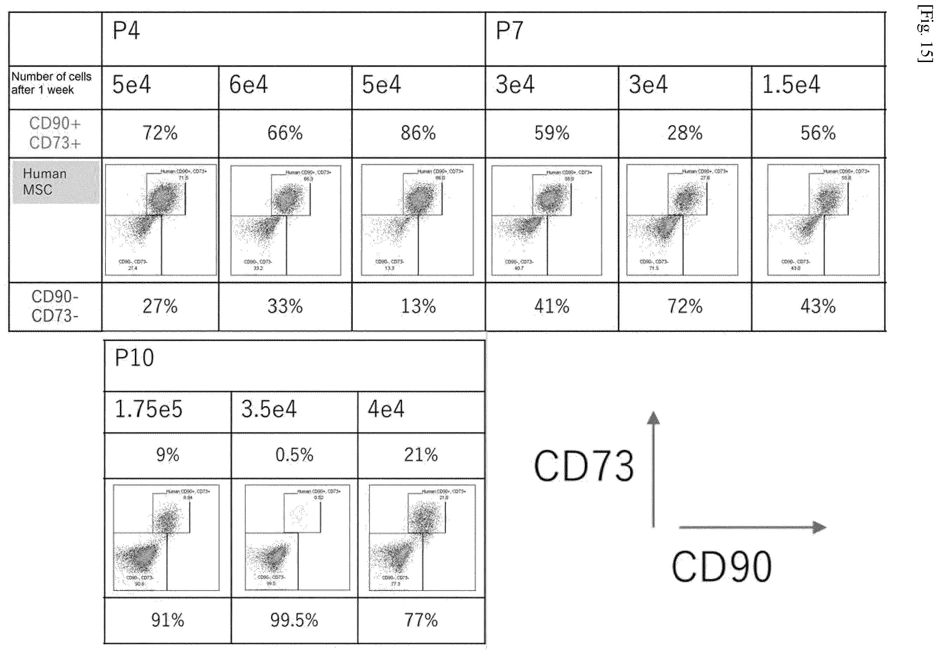

[FIG. 15] FIG. 15 shows a diagram illustrating the results of culture (reculture) of transplanted cells collected after transplantation of mesenchymal stem cells (Cellartis (TAKARA)) in the viability test (Example 5) for the mesenchymal stem cells in the animal performed with the passage number being varied. As shown in FIG. 15, the proliferated mesenchymal stem cells made up about 75% at the fourth passage (P4), whereas the proliferated mesenchymal stem cells made up about 48% at the seventh passage (P7), and the proliferated mesenchymal stem cells made up about 10% at the tenth passage (P10).

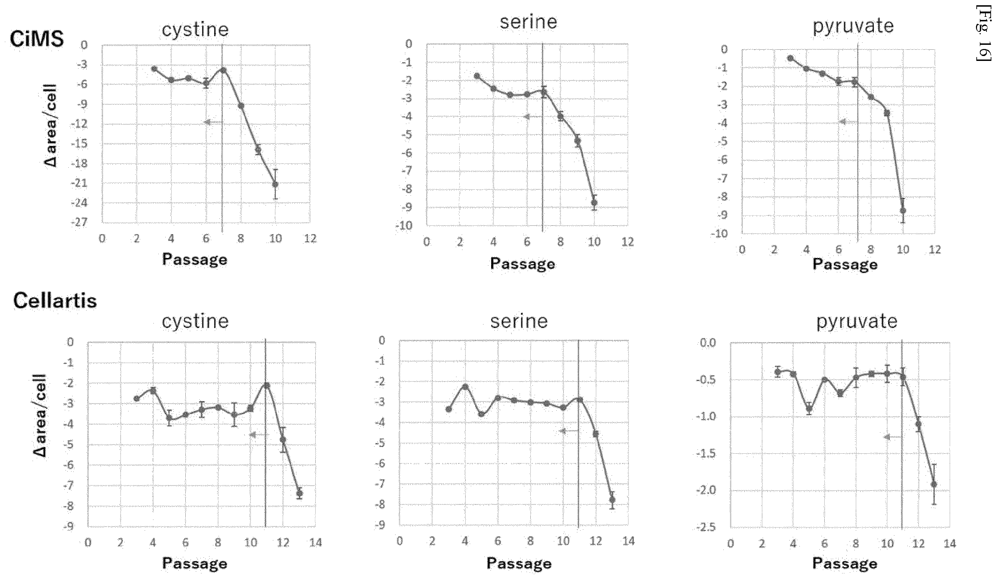

[FIG. 16] FIG. 16 shows diagrams illustrating the analysis results of consumptions of pyruvic acid, cystine, and serine per cell at passages (the third passage (P3), the fourth passage (P4), the fifth passage (P5), the sixth passage (P6), the seventh passage (P7), the eighth passage (P8), the ninth passage (P9), the tenth passage (P10), the eleventh passage (P 11), the twelfth passage (P12), and the thirteenth passage (P13)) when mesenchymal stem cells were cultured in CiMS/Cellartis. When CiMS was used (the three graphs in the right column in FIG. 16), the consumptions of pyruvic acid, cystine, and serine all increased after P7, and the consumptions of these three substances all increased in P8 to P10 as well. When Cellartis was used (the three graphs in the left column in FIG. 16), the consumptions of pyruvic acid, cystine, and serine all increased after P 11, and the consumptions of these three substances all increased in P12 and P13 as well.

### DESCRIPTION OF EMBODIMENTS

### 1. Method for Evaluating Quality of Mesenchymal Stem Cells According to the Present Invention (Mitochondrial Size, etc.)

The present invention relates to a method for evaluating quality of mesenchymal stem cells, the method including a step of evaluating the cells based on measurements of mitochondrial sizes in the cells.

More specifically, an aspect of the present invention is a method for evaluating quality of mesenchymal stem cells, the method including a step of evaluating the cells based on measurements of mitochondrial sizes in one cell.

Also, another aspect is a method for evaluating quality of mesenchymal stem cells, the method including a step of evaluating the cells based on the percentage of cells that have a size larger than or equal to a certain size and have mitochondria with a size larger than or equal to a particular size, with respect to total cells.

The "mitochondrial size" as used herein may be an absolute value or a relative value (relative to some reference value) as long as it can directly or indirectly indicate the sizes of mitochondria in the mesenchymal stem cells and can be used to evaluate the quality of the mesenchymal stem cells.

Specific examples of the "mitochondrial size" include the total volume and the total area of mitochondria in one cell. The total volume or the total area of mitochondria in a single cell may be a value obtained by measuring the total volume and the total area of mitochondria in particular one cell, or an average value of values obtained by measuring the total volumes and the total areas of mitochondria in a particular cell population (e.g., all the cultured cells or a portion of the cultured cells). Another specific example of the "mitochondrial size" is a value obtained by dividing the total volume or the total area of mitochondria in one cell by the size (e.g., the volume, the area, or the like) of the cell. Accordingly, an aspect of the present invention relates to a method for evaluating quality of mesenchymal stem cells, the method including a step of evaluating the cells based on measurements of mitochondrial sizes in one cell.

When directly measured, the volumes or areas (or the total volume or area) of mitochondria or cells may be measured (computed) based on images of the cells or mitochondria captured by, for example, a fluorescence microscope or confocal laser microscope (which will be described later) after staining of the mitochondria and the like with a fluorescent dye or the like (which will be also described later). When images of the cells are captured, one image per cell may be captured, or images of a plurality of divided portions of one cell may be captured. When images of a plurality of divided portions are captured, all or a portion of the images may be used to measure (compute) the volumes or areas (or the total volume or area) of the mitochondria or cells. The saving format of the captured images is not particularly limited as long as it can be used in an ordinary image analysis, and examples thereof include the TIFF format, the GIF format, the PNG format, and the JPEG format.

In addition to the above-described specific examples, another specific example of the "mitochondrial size" is fluorescence intensity obtained by staining cells or mitochondria with a fluorescent dye or the like and measuring the stained portions. A mitochondrion is an organelle that produces energy (ATP), and has membrane potential formed by proton gradient generated through oxidation-reduction reaction. Accordingly, when a fluorescent dye capable of staining mitochondria in a membrane potential-dependent manner (i.e., in a charge amount-dependent manner) is used, a measured fluorescence intensity can be used as a relative indicator of the mitochondrial size.

The membrane potential of a mitochondrion can be evaluated by measuring an electric potential difference between the outer mitochondrial membrane and the inner mitochondrial membrane, which is also used to produce ATPs. The membrane potential can be measured using a known reagent or the like for detecting mitochondrial membrane potential. The reagent may be, for example, a fluorescence reagent, and more specifically JC-1 (DOJINDO LABORATORIES), AIE Mitochondria Red (Funakoshi Co., Ltd.), or the like. A fluorescence microscope (e.g., BZ-X series; KEYENCE CORPORATION), a confocal laser microscope (e.g., VK-X series; KEYENCE CORPORATION), a (two-dimensional or three-dimensional) high-content confocal imaging system (e.g., Operetta CLS; PerkinElmer, Inc., Cell Voyger CV8000; Yokogawa Electric Corporation), a (microplate) fluorescence spectrophotometer (e.g., FP series; JASCO Corporation), flowcytometry (FACS (registered trademark); BD Biosciences), and the like can be used in the detection method.

In addition to the above-described specific examples, a specific example of the "mitochondrial size" is the percentage of cells that have a size larger than or equal to a predetermined size and have mitochondria with a size larger than or equal to a predetermined mitochondrial size (e.g., the above-described fluorescence intensity, total volume, total area, or the like of the mitochondria), with respect to a particular cell population (e.g., all the cultured cells or a portion of the cultured cells). Accordingly, another aspect of the present invention is a method for evaluating quality of mesenchymal stem cells, the method including a step of evaluating the cells based on the percentage of cells that have a size larger than or equal to a predetermined size and have mitochondria with a size larger than or equal to a predetermined size, with respect to a particular cell population.

The number, percentage, or the like of mesenchymal stem cells in a (cultured) cell population that are used in the evaluation method of the present invention can be obtained using a method known per se. Specifically, the number, percentage, or the like can be determined through flow cytometry or the like using a surface antigen (e.g., CD73, CD90, or CD105) of mesenchymal stem cells as an indicator.

The "step of evaluating the cells based on measurements of mitochondrial sizes in the cells" of the present invention is a step of determining whether or not the cells fall within the mitochondrial size range serving as a predetermined reference (value) (or cutoff value). A predetermined reference value (or cutoff value) for the mitochondrial size is, for example, as follows.

When mitochondria are stained with a fluorescent dye and the fluorescence intensity is measured using, for example, the same method as that in Examples, which will be described later, a predetermined reference value (or cutoff value) for the average value of the mitochondrial sizes in cells with a size of 100 K or more or cells with a diameter of (about) 30 µm or more may be 3300, 3345, 3400, 3500, 3568, 3600, 3700, 3800, 3900, 4000, 4100, 4200, 4300, 4400, 4500, 4600, 4616, 4700, 4800, 4900, 4924, or 5000. When the fluorescence intensity or the average value of the fluorescence intensities of mesenchymal stem cells or a mesenchymal stem cell population used in the evaluation method of the present invention is greater than or equal to the reference value (cutoff value) above, the cells or cell population can be determined to have poor quality and be unsuitable for, for example, medical applications (e.g., cell transplantation) and research applications.

Another predetermined reference value (cutoff value) may be a value obtained by comparing the fluorescence intensities above before and after a passage. "Comparing the fluorescence intensities before and after a passage" may be: (i) comparing the fluorescence intensity at a passage when the evaluation method of the present invention is conducted, with the fluorescence intensity at a predetermined reference passage (e.g., zeroth, first, second, or third passage); or (ii) comparing the fluorescence intensity at the n^{th} passage when the evaluation method of the present invention is conducted, with the fluorescence intensity at the n-1^{th} passage, where n-1 is the reference passage number. In this case, a predetermined reference value (cutoff value) is a value (after passage / before passage) that is obtained by dividing the fluorescence intensity or the average value of the fluorescence intensities after the passage by that before the passage, and that indicates that the fluorescence intensity or the average value of the fluorescence intensities tends to increase after the passage compared with before the passage, and may be, for example, 1.1 times, 1.2 times, 1.3 times, 1.4 times, 1.5 times, 1.6 times, 1.7 times, 1.8 times, 1.9 times, 2.0 times, 2.1 times, 2.2 times, 2.3 times, 2.4 times, 2.5 times, 2.6 times, 2.7 times, 2.8 times, 2.9 times, or 3.0 times. When the reference value (cutoff value) for the fluorescence intensity or the average value of the fluorescence intensities of mesenchymal stem cells or a mesenchymal stem cell population used in the evaluation method of the present invention reaches or exceeds the value above, the cells or cell population can be determined to have poor quality and be unsuitable for, for example, medical applications (e.g., cell transplantation) and research applications.

Regarding the above-described reference value (cutoff value), when a value obtained by comparing the fluorescence intensities before and after the passage is greater than or equal to the above-described reference value (cutoff value) two or more times in a row (e.g., the evaluation method of the present invention is conducted at the n^{th} passage and the n+1^{th} passage), the cells or cell population may be determined to have poor quality and be unsuitable for, for example, medical applications (e.g., cell transplantation) and research applications.

When, for example, the same method as that in Examples, which will be described later, is used to measure the percentage of cells that have a size larger than or equal to a predetermined size (90 K, 120 K, or the like) and have mitochondria with a fluorescence intensity (charge) larger than or equal to a predetermined fluorescence intensity (charge) (e.g., 2.5 K, 5.0 K, or the like) with respect to a particular cell population (e.g., all the cultured cells or a portion of the cultured cells), a reference value (cutoff value) therefor may be 10.0%, 10.4%, 10.7%, 11.0%, 12.0%, 13.0%, 13.2%, 14.0%, 15.0%, 15.4%, 16.0%, 17.0%, 18.0%, 19.0%, 20.0%, 21.0%, 22.0%, 22.6%, 23.0%, 24.0%, 25.0%, 26.0%, 26.3%, 27.0%, 28.0%, 29.0%, or 30.0%. When the percentage of cells that have a size larger than or equal to a predetermined size and have mitochondria with a fluorescence intensity (charge) larger than or equal to a predetermined fluorescence intensity (charge) with respect to a particular cell population (e.g., all the cultured cells or a portion of the cultured cells) used in the evaluation method of the present invention is greater than or equal to the reference value (cutoff value) above, the cells or cell population can be determined to have poor quality and be unsuitable for, for example, medical applications (e.g., cell transplantation) and research applications.

The evaluation method of the present invention may include a step of selecting cells with a coefficient of variation, a CV (Coefficient of Variation) value, smaller than or equal to a predetermined value in addition to the steps described above. The "CV value" as used herein is a value obtained by dividing a standard deviation by an average value. When, for example, the same method as that in Examples, which will be described later, is used to measure the CV value of cells or a cell population used in the evaluation method of the present invention, a reference value (cutoff value) therefor may be 40.0, 41.0, 42., 43.0, 43.5, 44.0, 45.0, 46.0, 46.4, 47.0, 48.0, 49.0, 50.0, 50.5, 51.0, 52.0, 52.2, 53.0, 53.1, 54.0, 54.8, 55.0, 55.3, 56.0, 56.6, 57.0, 58.0, 59.0, 60.0, 61.0, 62.0, 63.0, 64.0, 64.3, 65.0, 66.0, 67.0, 68.0, 69.0, or 70.0. Selecting cells or a cell population with a CV value smaller than or equal to the reference value (cutoff value) above (or removing cells or a cell population with a CV value larger than or equal to the reference value (cutoff value) above) makes it possible to more reliably select cells or a cell population that has good quality and is suitable for medical applications (e.g., cell transplantation), research applications, and the like.

The evaluation method of the present invention may include a step of selecting cells with a mesenchymal stem cell proliferation ratio larger than or equal to a predetermined value in addition to the steps described above. When, for example, the same method as that in Examples, which will be described later, is used to measure the proliferation ratio of cells or a cell population used in the evaluation method of the present invention, a reference value (cutoff value) therefor may be 5 times, 6 times, 7 times, 8 times, 9 times, 10 times, 11 times, 12 times, 13 times, 14 times, or 15 times. Selecting cells or a cell population with a proliferation ratio larger than or equal to the reference value (cutoff value) above (or removing cells or a cell population with a CV value smaller than or equal to the reference value (cutoff value) above) makes it possible to more reliably select cells or a cell population that has good quality and is suitable for medical applications (e.g., cell transplantation), research applications, and the like.

In the present specification, "cells" may include "cell population" unless otherwise stated. The cell population may be composed of one type of cell or two or more types of cells.

Although there is no particular limitation on a source species of mesenchymal stem cells used in the evaluation method of the present invention, examples of the source species include rodents such as a rat, a mouse, a hamster, and a guinea pig, lagomorphs such as rabbit, ungulates such as a pig, a cow, a goat, and a sheep, carnivores such as a dog and a cat, and primates such as a human, a monkey, a rhesus monkey, a marmoset, an orangutan, and a chimpanzee.

### 2. Method for Producing Mesenchymal Stem Cells According to the Present Invention (Mitochondrial Size, etc.)

The present invention relates to a method for producing mesenchymal stem cells evaluated for quality, the method including a step of evaluating the cells (a step of evaluating quality) based on measurements of mitochondrial sizes in the cells, and also relates to mesenchymal stem cells obtained using this production method. More specifically, the method for producing mesenchymal stem cells according to the present invention includes: (A) a step of preparing mesenchymal stem cells to be subjected to expansion culture; and (B) a step of conducting expansion culture of the prepared cells, and the step of evaluating quality may be conducted before the step (A), during the step (A), after the step (A), during the step (B), or after the step (B). The step of evaluating quality may be conducted once in a particular period or a plurality of times. In an aspect of the present invention, the present invention includes: (1) a step of preparing mesenchymal stem cells evaluated for quality; and (2) a step of culturing the mesenchymal stem cells.

Although there is no particular limitation on a source species of cells that can be used in the production method of the present invention, examples of the species include rodents such as a rat, a mouse, a hamster, and a guinea pig, lagomorphs such as rabbit, ungulates such as a pig, a cow, a goat, and a sheep, carnivores such as a dog and a cat, and primates such as a human, a monkey, a rhesus monkey, a marmoset, an orangutan, and a chimpanzee. Also, there is no particular limitation on the age and the sex of the source species. When mesenchymal stem cells are produced using the production method of the present invention for the purpose of administration to humans, it is preferable to use, as a material, a cell population collected from a donor in which the type of histocompatibility antigen is the same as or similar to that of a recipient. It is more preferable to subject a cell population collected from a recipient himself/herself to production of mesenchymal stem cells.

Mesenchymal stem cells that are prepared and subjected to expansion culture in the production method of the present invention may be primary cells that are directly separated from a biological tissue containing mesenchymal stem cells, or mesenchymal stem cells differentiated or induced from an established mesenchymal stem cell strain, embryonic stem cells, or induced pluripotent stem cells, or cryopreserved products of these cells. Here, the term "directly" means that an in-vitro culturing/proliferation step is not involved.

As a method for obtaining mesenchymal stem cells directly separated from a biological tissue containing mesenchymal stem cells, a method known per se such as the method disclosed in WO 2017/094879 is used to obtain desired cells.

Examples of the above-described biological tissue containing mesenchymal stem cells include a bone marrow, an adipose tissue, blood, a placenta, an umbilical cord, and a dental pulp. Out of the tissues above, the adipose tissue is a preferable source for collecting the cells above because the adipose tissue can be collected through liposuction or adipose tissue excision and this collection method is less likely to cause dysfunction in the living body.

An adipose tissue is one type of biological tissue composed of adipocytes. When the adipose tissue is used as a collection source of the cells above, there is no particular limitation on the site of the adipose tissue, but examples thereof include subcutaneous fat, visceral fat, intramuscular fat, and intermuscular fat. Out of these, subcutaneous fat can be said to be preferable because it can be easily collected under local anesthesia and burden on a donor during the collection can be reduced.

Although there is no particular limitation on a method for preparing an adipose tissue, the adipose tissue can be prepared from a tissue fragment sucked through liposuction surgery during cosmetic surgery or from an excised adipose tissue contained in a tissue excised from a living body during a surgical operation or the like. Fat-derived stem cells are present around thick blood vessels, and thus more fat-derived stem cells can be obtained from an excised adipose tissue than from a fat aspirate. On the other hand, when stem cells are prepared from a fat aspirate, the size of an operation scar can be made smaller, and burden on a donor can be reduced. Note that one type of adipose tissue is commonly used, but two or more types of adipose tissues may be used together. Also, adipose tissues collected a plurality of times may be mixed and used.

The collection amount of the adipose tissue can be determined as appropriate in consideration of the type of donor, the type of tissue, or the amount of required mesenchymal stem cells. For example, mesenchymal stem cells can be obtained from 0.3 to 20 g of the adipose tissue, but the present invention is not limited thereto. A larger amount of the adipose tissue can be used as a starting material through a scale-up or by performing the operation a plurality of times. The collected adipose tissue is subjected to removal of blood components attached thereto and fragmentation as needed, and is then subjected to enzyme treatment (protease treatment), which will be described later. Note that the blood components can be removed by washing the adipose tissue in an appropriate buffer solution or culture solution.

The enzyme treatment is conducted by digesting the adipose tissue with a protease such as collagenase, trypsin, or Dispase. This enzyme treatment can be conducted using, for example, a method known per se such as the method described in R. Ian Freshney, Culture of Animal Cells: A Manual of Basic Technique, Fourth Edition, A John Wiley & Sones Inc., Publication.

The enzyme-treated adipose tissue includes two principal cell populations (stromal vascular fraction and mature adipocytes). The stromal vascular fraction is a cell mixture containing preadipocytes, mature endothelial cells, endothelial precursor cells, vascular smooth muscle cells, pericytes, wall cells, macrophages, fibroblasts, and fat-derived stem cells. Fat-derived stem cells are mesenchymal stem cells that can easily differentiate into adipocytes, osteoblasts, chondrocytes, and the like. The types and ratios of cells included in the cell population depend on the source and type of the adipose tissue used.

The enzyme-treated adipose tissue is then subjected to centrifugation and is thus separated into two cell populations, and the precipitate (that includes the stromal vascular fraction) is collected. Although the conditions of the centrifugation vary depending on the types and amounts of the cells, the centrifugation is conducted, for example, at 300 to 2000×g for 1 to 15 minutes. Prior to the centrifugation, the enzyme-treated cell population may be subjected to filtration or the like to remove the tissue that has not been undigested by the enzyme, and the like from the cell population. For example, a filter with a pore diameter of 50 to 2000 µm and preferably a filter with a pore diameter of 100 µm can be preferably used for the filtration

Mesenchymal stem cells can be selectively proliferated by culturing the above-described stromal vascular fraction containing mesenchymal stem cells in the presence of a fibronectin fragment in accordance with a method known per se such as the method disclosed in WO 2017/094879. The mesenchymal stem cells proliferated as described above (corresponding to mesenchymal stem cells prepared in the step (A) of the production method of the present invention) may be used in the expansion culture step (B) of the production method of the present invention.

### (i) Proliferation Culture for Preparation of Mesenchymal Stem Cells

Mesenchymal stem cells prepared in the step (A) of the production method of the present invention may be produced as follows. A medium for selective proliferation of mesenchymal stem cells can be prepared using, as a basal medium, a medium used to culture normal animal cells. A favorable example of the medium is a medium that does not contain a xenogeneic component such as fetal bovine serum (FBS) (or fetal calf serum (FCS)) or sheep serum. Although such a xenogeneic component-free (xeno-free) medium can be prepared as appropriate, a medium known per se or a commercially available medium may be used as it is or after being modified. Examples of the commercially available xeno-free medium include Cellartis (registered trademark) DEF-CS500 XF (TAKARA), Mesenchymal Stem Cell Growth Medium DXF (PromoCell), CiMS-BM (NIPRO), StemPro MSC SFM XenoFree (Thermo Fisher Scientific), and KBM ADSC-4 (KOHJIN BIO). These media may also be employed as a medium to be used in the expansion culture step (B) of the production method of the present invention, which will be described later.

Additives known per se can be added to the medium. There is no particular limitation on the additives as long as they do not inhibit the proliferation of mesenchymal stem cells, and examples thereof include growth factors (e.g., insulin), iron sources (e.g., transferrin), polyamines (e.g., putrescine), minerals (e.g., sodium selenate), saccharides (e.g., glucose), organic acids (e.g., pyruvic acid and lactic acid), amino acids (e.g., L-glutamine), reducing agents (e.g., 2-mercaptoethanol), vitamins (e.g., ascorbic acid and d-biotin), steroids (e.g., β-estradiol and progesterone), antibiotics (e.g., streptomycin, penicillin, and gentamicin), buffering agents (e.g., HEPES), lipids (e.g., linoleic acid), and nucleic acids (e.g., thymidine). Also, additives known per se that have been conventionally used for culture of mesenchymal stem cells may be added as appropriate. It is preferable that the concentrations of the contained additives are within ranges known per se. These additives may also be employed as additives for a medium to be used in the expansion culture step (B) of the production method of the present invention, which will be described later.

When mesenchymal stem cells are produced from an autologous tissue for the purpose of administration to a living body, the medium may contain allogeneic serum or may be a serum-free medium. Preferably, for example, the medium is an allogeneic serum-containing medium or serum-free medium and contains no xenogeneic components. The allogeneic serum is preferably autologous serum. Here, the "autologous serum" and "autologous plasma", which will be described later, respectively mean serum and plasma obtained from the blood collected from a donor who is identical to a donor of a cell population to be cultured.

Since the plasma contains serum components, a medium containing autologous plasma may be used. Preferably, inactivated autologous plasma is added to the medium. For example, cells are cultured in a culture solution containing inactivated autologous plasma at a concentration of 10% (V/V) or less, preferably 5% (V/V) or less, and more preferably 2% (V/V) or less. Using autologous plasma makes it possible to exclude xenogeneic components from the production process, and thus highly safe cells can be produced. The serum and the plasma may also be employed as serum and plasma for a medium to be used in the expansion culture step (B) of the production method of the present invention, which will be described later.

Cell culture conditions can be set as appropriate. For example, the culture temperature is not particularly limited but can be about 30 to 40°C and preferably about 37°C. The CO₂ concentration can be about 1 to 10% and preferably about 2 to 5%. The oxygen concentration can be 1 to 20% and preferably 1 to 10%. It is preferable to replace the medium containing an active component at appropriate intervals during the culture. These culture conditions may also be employed as culture conditions for the expansion culture step (B) of the production method of the present invention, which will be described later.

There is no particular limitation on the culture vessel used for the culture of mesenchymal stem cells as long as mesenchymal stem cells can be cultured therein, and examples thereof include a flask, a tissue culture flask, a dish, a petri dish, a tissue culture dish, a multi-dish, a microplate, a microwell plate, a multi-plate, a multi-well plate, a microslide, a chamber slide, a laboratory dish, a tube, a tray, a culture bag, and a roller bottle. These culture vessels may also be employed as culture vessels to be used in the expansion culture step (B) of the production method of the present invention, which will be described later.

As described above, in an aspect of the production method of the present invention, cells may be cultured, for example, in the presence of a fibronectin fragment as described in WO 2017/094879 (e.g., RetroNectin (registered trademark) (Takara Bio Inc.)) containing a functional domain of fibronectin, that is, a cell adhesion domain or a heparin-binding domain. A specific culturing method is as follows: a stromal vascular fraction containing mesenchymal stem cells is cultured for, for example, 4 to 14 days and preferably 7 to 10 days using a xenogeneic component-free medium supplemented with 5% autologous plasma in a vessel coated with a fibronectin fragment with the medium being replaced every 3 to 4 days. This culture enables selective proliferation of mesenchymal stem cells. Mesenchymal stem cells make up 80% or more and preferably 90% or more of the cell population obtained through this culture. This culture method conducted in the presence of a fibronectin fragment may also be employed as a culture method to be used in the expansion culture step (B) of the production method of the present invention, which will be described later.

In isolation culture, the cell concentration at the start of the culture is not particularly limited, but is, for example, 0. 1 to 10×10⁵ cells/ml, preferably 0.3 to 5×10⁵ cells/ml, and more preferably 0.5 to 2×10×10⁵ cells/ml. These cell concentrations may also be employed as a cell concentration in the expansion culture step (B) of the production method of the present invention, which will be described later.

### (ii) Expansion Culture of Mesenchymal Stem Cells

Expansion culture of mesenchymal stem cells (step (B) of the production method of the present invention) may be conducted using a method known per se as long as the desired number of cells with excellent quality can be secured. For example, in an aspect, cells may be cultured using a medium for mesenchymal stem cells containing reduced amounts of particular non-essential amino acids (glycine, alanine, serine, proline, asparagine, aspartic acid, and glutamic acid), as described in WO 2016/027850. Also, in another aspect, cells may be cultured in the presence of a fibronectin fragment as described in WO 2017/094879 (e.g., RetroNectin (registered trademark) (Takara Bio Inc.)) containing a functional domain of fibronectin, that is, a cell adhesion domain or a heparin-binding domain.

In an aspect of the production method of the present invention, when, for example, mesenchymal stem cells and the like directly separated from the above-described biological tissue containing mesenchymal stem cells are used in the expansion culture step (B) of the production method of the present invention, the expansion culture may be conducted using a method as follows.

The expansion culture step (B) is a step of extensively proliferating mesenchymal stem cells through expansion culture of mesenchymal stem cells in the presence of a fibronectin fragment. Examples of the mesenchymal stem cells subjected to the culture of this step (B) include mesenchymal stem cells produced as described in "Proliferation Culture for Preparation of Mesenchymal Stem Cells" above, and mesenchymal stem cells differentiated or induced from an established mesenchymal stem cell strain, embryonic stem cells, and induced pluripotent stem cells.

A method known per se may be used to separate the mesenchymal stem cells produced as described in "Proliferation Culture for Preparation of Mesenchymal Stem Cells" above from the culture vessel. Specific examples of the method include a physical method, a method in which a chelating agent is used, an enzymatic method in which a separating liquid having protease activity and/or collagenase activity (e.g., Accutase (registered trademark) and Accumax (registered trademark) (both available from Innovative Cell Technologies, Inc.), and the like) is used, and combinations thereof. In a preferable example of the method, a sheet of mesenchymal stem cells is separated using an enzymatic method, and then the cells are finely dispersed using a physical method. Here, it is preferable to use cells cultured to 70 to 95% confluency with respect to the culture vessel used, and it is more preferable to use cells cultured to 80 to 90% confluency.

The separated cells may be used as they are, or may be cryopreserved until use. Also, a subset of cells separated based on a particular cell surface marker, such as isolated mesenchymal stem cells, may be used in this step.

The same medium and the same culture conditions as those described in "Proliferation Culture for Preparation of Mesenchymal Stem Cells" above can be employed in the expansion culture step (B). Similarly to the culture described in "Proliferation Culture for Preparation of Mesenchymal Stem Cells" above, it is preferable to use a medium containing no xenogeneic components, and any xenogeneic component-free medium such as a known medium or a commercially available medium may be used as it is or after being modified.

In an aspect of the expansion culture step (B), expansion culture of the mesenchymal stem cells cultured through the proliferation culture for preparation of mesenchymal stem cells is conducted using a xenogeneic component-free medium containing no serum in a vessel coated with a fibronectin fragment for, for example, 4 to 14 days and preferably 6 to 12 days with the medium being replaced every 3 to 4 days while the cells are passaged as appropriate. This culture enables extensive proliferation of mesenchymal stem cells.

In the expansion culture step (B), the cell concentration at the start of the culture is not particularly limited as long as mesenchymal stem cells can be proliferated, but it is typically, for example, 1.0×10¹ to 1.0×10⁶ cells/cm², preferably 1.0×10² to 1.0×10⁵ cells/cm², more preferably 1.0×10³ to 1.0×10⁵ cells/cm², even more preferably 5.0×10³ to 5.0×10⁴ cells/cm².

Mesenchymal stem cells can be obtained using the above-described method for producing mesenchymal stem cells according to the present invention. The mesenchymal stem cells obtained using the production method of the present invention can be confirmed by detecting a molecule (e.g., an enzyme, a receptor, or a low-molecular compound) characteristic of mesenchymal stem cells. Examples of the molecule characteristic of mesenchymal stem cells include CD73, CD90, CD105, and CD166, which are cell surface markers (positive markers), but there is no limitation thereto. Also, CD19, CD34, CD45, HLA-DR, CD11b, CD14, and the like are known as negative markers, which are not expressed in mesenchymal stem cells, and can be used for confirmation of mesenchymal stem cells. Although an immunological method can be used to detect the molecules above, the molecules may be detected through quantification of the mRNA levels of the molecules.

For example, 80% or more, preferably 90% or more, and more preferably 95% or more of the mesenchymal stem cells obtained using the production method of the present invention express the positive marker above. Also, the negative marker expression ratio in the mesenchymal stem cells obtained using the production method of the present invention is, for example, 5% or less, preferably 1% or less, and more preferably lower than or equal to the detection limit.

The mesenchymal stem cells obtained using the production method of the present invention may be further isolated, separated, and purified using an antibody that recognizes the molecule characteristic of the cells.

### 3. Method for Producing Mesenchymal Cells According to the Present Invention (Mitochondrial Size, etc.)

The present invention relates to a method for producing mesenchymal cells (differentiated cells), the method including a step of inducing differentiation of mesenchymal stem cells obtained using the method for producing mesenchymal stem cells (mitochondrial size, etc.) according to the present invention, and also relates to mesenchymal cells obtained using this production method. More specifically, the present invention includes: (1) a step of preparing mesenchymal stem cells obtained using the method for producing mesenchymal stem cells according to the present invention; and (2) a step of inducing differentiation into the mesenchymal cells. Examples of such mesenchymal cells include osteocytes, chondrocytes, and adipocytes. It is preferable to conduct all the steps of the method for producing mesenchymal cells according to the present invention under the feeder-free and xeno-free conditions.

Examples of an osteocyte differentiation-inducing medium include a basal medium (e.g., αMEM) containing 10% FBS, 0.1 µM dexamethasone, 50 µg/ml ascorbic acid, and 10 mM β-glycerophosphate, and a commercially available xeno-free osteocyte differentiation-inducing medium (e.g., MSCgo (trademark) Rapid Osteogenic Differentiation Medium (Biological Industries)). Specifically, the osteocyte differentiation method is conducted as follows, for example: 4×10⁴ mesenchymal stem cells are seeded in a 12-well plate coated with gelatin and are cultured in the above-described osteocyte differentiation-inducing medium for 30 days. Differentiation into osteocytes may be confirmed by detecting calcified nodules through alizarin red staining.

Examples of a chondrocyte differentiation-inducing medium include a basal medium (e.g., DMEM/F12) containing 1%(v/v) ITS+ premix, 0.17 mM AA2P, 0.35 mM proline, 0.1 mM dexamethasone, 0.15% (v/v) glucose, 1 mM sodium pyruvate, 2 mM GlutaMAX, 40 ng/ml PDGF-BB, 100 ng/mL TGF-β3, 10 ng/ml BMP4, and 1% (v/v) FBS, and a commercially available xeno-free chondrocyte differentiation-inducing medium (e.g., MSCgo (trademark) Chondrogenic XF (Biological Industries)). Specifically, the chondrocyte differentiation method is conducted as follows, for example: 5 µl of a mesenchymal stem cell suspension is spotted on a plate coated with fibronectin, followed by 1-hour culture, and 1 ml of the above-described differentiation-inducing medium is added thereto after 1 hour, followed by 14-day culture. Differentiation into chondrocytes may be confirmed through alcian blue staining.

Examples of an adipocyte differentiation-inducing medium include a basal medium containing 60 µM indomethacin, 0.5 mM IBMX, and 0.5 µM hydrocortisone, and a commercially available xeno-free adipocyte differentiation-inducing medium (e.g., hMSC-Human Mesenchymal Stem Cell Adipogenic Differentiation Medium (Lonza) or MSCgo (treademark) Adipogenic XF (Biological Industries)). Specifically, the adipocyte differentiation method is conducted as follows, for example: 4×10⁴ mesenchymal stem cells are seeded on a plate coated with gelatin and are cultured in the above-described adipocyte differentiation-inducing medium for 32 days. Differentiation into adipocytes may be confirmed through oil red O staining.

The contents described in "2. Method for Producing Mesenchymal Stem Cells According to the Present Invention (Mitochondrial Size, etc.)" are applied to the other culture conditions, culturing method, additives for a medium, specific examples of the culture vessel, way to process the surface of the culture vessel, and the like.

### 4. Cell Transplantation Therapy Agent (Mitochondrial Size, etc.)

The mesenchymal stem cells obtained using the method for producing mesenchymal stem cells (mitochondrial size, etc.) according to the present invention and the mesenchymal cells (differentiated cells) obtained using the method for producing mesenchymal cells (mitochondrial size, etc.) according to the present invention (these cells may be collectively referred to as "mesenchymal cells of the present invention" hereinafter) can have excellent repairing ability when transplanted to a defect site of the bone, cartilage, muscle, or the like, compared with cells obtained using conventional methods. Accordingly, the mesenchymal cells of the present invention can be favorably used in cell transplantation therapy. Therefore, the present invention relates to a cell transplantation therapy agent containing the mesenchymal cells of the present invention. In addition, the present invention also encompasses a method for treating an injury (including defect) of a tissue (e.g., a bone tissue, a cartilage tissue, an adipose tissue, and a muscle tissue (e.g., skeletal muscle tissue)) or a disease, the method including administering or transplanting an effective amount of the mesenchymal cells of the present invention to a mammal (e.g., a human, a mouse, a rat, a monkey, a cow, a horse, a pig, or a dog) to be treated. Also, the "treatment of an injury of a tissue" encompasses regeneration of an injured tissue.

The purpose of the transplantation of the mesenchymal cells of the invention to a living body may be direct regeneration of an injured tissue or an indirect effect (e.g., paracrine effect) by a factor secreted by the mesenchymal cells of the present invention. For example, the therapeutic effect of mesenchymal stem cells can be obtained in patients affected with acute myocardial infarction, a stroke, multiple system atrophy (MSA), a graft-versus-host disease, Crohn's disease, ischemic cardiomyopathy, spinal cord injury, and the like.

When the mesenchymal cells of the present invention are used in cell transplantation therapy, it is desirable to use cells derived from an individual with the same or substantially the same HLA genotype as that of a transplantation target individual or cells derived from iPS cells established from somatic cells of the individual, from the viewpoint of preventing rejection. Here, "substantially the same" means that the HLA genotypes are identical to an extent that the immunological reaction to the transplanted cells can be suppressed using an immunosuppressive agent, and, for example, somatic cells with the HLA type in which three genetic loci (HLA-A, HLA-B, and HLA-DR) are respectively identical or four genetic loci (HLA-C in addition to the three loci above) are respectively identical are used. When a sufficient amount of cells cannot be obtained due to the age, diathesis, or the like, the cells can be embedded in a capsule made of polyethylene glycol or silicone, a porous vessel, or the like for the purpose of avoiding rejection and then transplanted.

A parenteral preparation such as an injection, a suspension, or a drip is produced by, for example, mixing the mesenchymal cells of the present invention with a pharmaceutically acceptable carrier in accordance with a conventional procedure. Accordingly, in an aspect, a method for producing a cell transplantation therapy agent is also provided, the method including a step of formulating the mesenchymal cells of the present invention. This production method may include a step of preparing the mesenchymal cells of the present invention. In addition, the production method can also include a step of preserving the mesenchymal cells of the present invention.

Examples of the pharmaceutically acceptable carrier that can be contained in the parenteral preparation include aqueous liquids for injection such as a physiological saline solution and an isotonic solution containing glucose and other adjuvants (e.g., D-sorbitol, D-mannitol, and sodium chloride). The cell transplantation therapy agent of the present invention may be blended with a buffering agent (e.g., a phosphate buffer solution or sodium acetate buffer solution), a soothing agent (e.g., benzalkonium chloride or procaine hydrochloride), a stabilizer (e.g., human serum albumin or polyethylene glycol), a preservative, an antioxidant, and the like. When the cell transplantation therapy agent of the present invention is formulated as an aqueous suspension, it is only necessary to suspend the cells into the above-described aqueous liquid such that, for example, the cell concentration is about 1×10⁶ to about 1×10⁸ cells/ml. In addition, the dosage or transplantation amount and the administration frequency or transplantation frequency of the mesenchymal cells or pharmaceutical composition of the present invention can be determined as appropriate depending on the age, weight, symptom, and the like of a mammal to which the mesenchymal cells or pharmaceutical composition is administered.

The cell transplantation therapy agent of the present invention can be used as follows: it is provided in a state of being cryopreserved under the conditions commonly used for cryopreservation of cells, and is thawed before use. In such a case, the cell transplantation therapy agent may further contain serum or a substitute thereof, an organic solvent (e.g., DMSO), and the like. In such a case, the concentration of the serum or the substitute thereof is not particularly limited, but can be about 1 to about 30% (v/v) and preferably about 5 to about 20% (v/v). The concentration of the organic solvent is not particularly limited, but can be 0 to about 50% (v/v) and preferably about 5 to about 20% (v/v).

### 5. Method for Evaluating Medium for Mesenchymal Stem Cells According to the Present Invention (Mitochondrial Size, etc.)

The present invention relates to a method for evaluating a medium for mesenchymal stem cells, the method including a step of evaluating the medium based on measurements of mitochondrial sizes in the cells.

More specifically, an aspect of the present invention relates to a method for evaluating a medium for mesenchymal stem cells (evaluation method 1 of the present invention), the method including:
(1) a step of culturing mesenchymal stem cells in a medium to be evaluated;
(2) a step of measuring mitochondrial sizes in the cultured mesenchymal stem cells; and
(3) a step of comparing the measured mitochondrial sizes in the cells with a predetermined reference value.

Another aspect relates to a method for evaluating a medium for mesenchymal stem cells (evaluation method 2 of the present invention), the method including:
(i) a step of measuring mitochondrial sizes in mesenchymal stem cells before the mesenchymal stem cells are cultured in a medium to be evaluated;
(ii) a step of culturing the mesenchymal stem cells in the medium to be evaluated;
(iii) a step of measuring mitochondrial sizes in the cultured mesenchymal stem cells; and
(iv) a step of comparing the mitochondrial sizes in the cells measured in the step (i) and the step (iii).

Specific examples of the mitochondrial size include those described in "1. Method for Evaluating Quality of Mesenchymal Stem Cells According to the Present Invention (Mitochondrial Size, etc.)" above. When mitochondria are stained with a fluorescent dye and the fluorescence intensity is measured using, for example, the same method as that in Examples, which will be described later, a predetermined reference value (or cutoff value) for the average value of the mitochondrial sizes in cells with a size of 100 K or more or cells with a diameter of (about) 30 µm or more may be 3300, 3345, 3400, 3500, 3568, 3600, 3700, 3800, 3900, 4000, 4100, 4200, 4300, 4400, 4500, 4600, 4616, 4700, 4800, 4900, 4924, or 5000 after a predetermined number of passages (e.g., 1, 2, 3, 4, 5, 6, 7, 8, 9, 10, 11, 12, 13, 14, 15, 16, 17, 18, 19, or 20 passages). When the fluorescence intensity or the average value of the fluorescence intensities of the mesenchymal stem cells or the mesenchymal stem cell population is greater than or equal to the reference value (cutoff value) above, the medium can be determined to be unsuitable as a medium for mesenchymal stem cells.

Another predetermined reference value (cutoff value) may be a value obtained by comparing the fluorescence intensities above before and after the culture. "Comparing the fluorescence intensities before and after the culture" may mean comparison of the fluorescence intensity before the start of the culture and the fluorescence intensity after a predetermined number of passages (e.g., 1, 2, 3, 4, 5, 6, 7, 8, 9, 10, 11, 12, 13, 14, 15, 16, 17, 18, 19, or 20 passages). In this case, a predetermined reference value (cutoff value) is a value (after passage / before passage) that is obtained by dividing the fluorescence intensity or the average value of the fluorescence intensities after the culture by that before the culture, and that indicates that the fluorescence intensity or the average value of the fluorescence intensities tends to increase after the culture compared with before the culture, and may be, for example, 1.1 times, 1.2 times, 1.3 times, 1.4 times, 1.5 times, 1.6 times, 1.7 times, 1.8 times, 1.9 times, 2.0 times, 2.1 times, 2.2 times, 2.3 times, 2.4 times, 2.5 times, 2.6 times, 2.7 times, 2.8 times, 2.9 times, or 3.0 times. When the reference value (or cutoff value) for the fluorescence intensity or the average value of the fluorescence intensities of the mesenchymal stem cells or the mesenchymal stem cell population used in the evaluation method of the present invention reaches or exceeds the value above, the medium can be determined to be unsuitable as a medium for mesenchymal stem cells.

When the percentage of cells that have a size larger than or equal to a predetermined size (90 K, 120 K, or the like) and have mitochondria with a fluorescence intensity (charge) larger than or equal to a predetermined fluorescence intensity (charge) (e.g., 2.5 K, 5.0 K, or the like), with respect to a particular cell population (e.g., all the cultured cells or a portion of the cultured cells) is employed and is measured using, for example, the same method as that in Examples, which will be described later, a reference value (or cutoff value) therefor may be 10.0%, 10.4%, 10.7%, 11.0%, 12.0%, 13.0%, 13.2%, 14.0%, 15.0%, 15.4%, 16.0%, 17.0%, 18.0%, 19.0%, 20.0%, 21.0%, 22.0%, 22.6%, 23.0%, 24.0%, 25.0%, 26.0%, 26.3%, 27.0%, 28.0%, 29.0%, or 30.0% after a predetermined number of passages (e.g., 1, 2, 3, 4, 5, 6, 7, 8, 9, 10, 11, 12, 13, 14, 15, 16, 17, 18, 19, or 20 passages). When the percentage of cells that have a size larger than or equal to a predetermined size and have mitochondria with a fluorescence intensity (charge) larger than or equal to a predetermined fluorescence intensity (charge) with respect to a particular cell population (e.g., all the cultured cells or a portion of the cultured cells) after a predetermined number of passages is greater than or equal to the reference value (cutoff value) above, the medium can be determined to be unsuitable as a medium for mesenchymal stem cells.

In the method for evaluating a medium for mesenchymal stem cells according to the present invention, a coefficient of variation, a CV (Coefficient of Variation) value, indicating the variation in the cell size may be used as a reference value in addition to the above-described reference value (cutoff value). The "CV value" as used herein is a value obtained by dividing a standard deviation by an average value. When the CV value measured using the same method as that in Examples, which will be described later, a predetermined reference value (or cutoff value) therefor may be 40.0, 41.0, 42.0, 43.0, 43.5, 44.0, 45.0, 46.0, 46.4, 47.0, 48.0, 49.0, 50.0, 50.5, 51.0, 52.0, 52.2, 53.0, 53.1, 54.0, 54.8, 55.0, 55.3, 56.0, 56.6, 57.0, 58.0, 59.0, 60.0, 61.0, 62.0, 63.0, 64.0, 64.3, 65.0, 66.0, 67.0, 68.0, 69.0, or 70.0 after a predetermined number of passages (e.g., 1, 2, 3, 4, 5, 6, 7, 8, 9, 10, 11, 12, 13, 14, 15, 16, 17, 18, 19, or 20 passages). Selecting a medium from which cells or a cell population with a CV value smaller than or equal to the reference value (cutoff value) above is obtained after a predetermined number of passages (or removing a medium) with which cells or a cell population with a CV value larger than or equal to the reference value (cutoff value) above is obtained makes it possible to more reliably select a medium suitable as a medium for mesenchymal stem cells.

All the contents described in "1. Method for Evaluating Quality of Mesenchymal Stem Cells According to the Present Invention (Mitochondrial Size, etc.)", "2. Method for Producing Mesenchymal Stem Cells According to the Present Invention (Mitochondrial Size, etc.)", and "3. Method for Producing Mesenchymal Cells According to the Present Invention (Mitochondrial Size, etc.)" are applied to the matters required to conduct the method for evaluating a medium for mesenchymal stem cells according to the present invention other than those described above.

### 6. Method for Evaluating Quality of Mesenchymal Stem Cells According to the Present Invention (Consumptions of Pyruvic Acid, Cystine, and Serine, etc.)

The present invention relates to a method for evaluating quality of mesenchymal stem cells, the method including a step of evaluating the cells based on measurements of consumptions of pyruvic acid, cystine, and serine by the cells.

More specifically, an aspect of the present invention relates to a method for evaluating quality of mesenchymal stem cells, the method including a step of evaluating the cells based on measurements of consumptions of pyruvic acid, cystine, and serine by one cell.

In the present invention, the "consumptions of pyruvic acid, cystine, and serine by the cells" can be calculated based on differences in abundances of pyruvic acid, cystine, and serine in the medium between before and after the start of the culture of mesenchymal stem cells. "Before the start of the culture" as used herein may mean "before the start of the first passage (maintenance) culture (or at the time when the first passage (maintenance) culture is started) (P0)", or "before the start of any of the passages (e.g., P1, P2, P3, P4, P5, P6, P7, P8, P9, P10, P11, P12, P13, P14, or P15) (or at the time when any of the passages is started)". Also, "after the start of the culture" as used herein may mean "after the end of any of the passages (e.g., P1, P2, P3, P4, P5, P6, P7, P8, P9, P 10, P11, P12, P13, P14, or P 15) (or at the time when any of the passages is finished)". The abundances of pyruvic acid, cystine, and serine in the medium may be represented by the concentrations, or the measurement values obtained by measuring these three substances using an apparatus or a method as described later. In the present invention, the "differences in abundances" may by differences in concentrations of pyruvic acid, cystine, and serine in the medium between before and after the start of the culture, or differences in measurement values as described above. There is no particular limitation on a method and an apparatus for measuring the abundances of pyruvic acid, cystine, and serine in the medium as long as the three substances can be measured with desired detection limits, and the abundances thereof can be measured using a method known per se.

Specifically, for example, a liquid chromatograph/tandem mass spectrometer (LC/MS/MS), a liquid chromatograph/mass spectrometer (LC/MS), an immunological measurement method (e.g., ELISA) can be used. In this measurement, these three substances may be simultaneously measured using the same apparatus or method, or may be separately measured using the same apparatus or method, or may be separately measured using different apparatuses or methods.

In an aspect of the present invention, the "consumptions of pyruvic acid, cystine, and serine by the cells", which are used as indicators in the present invention, can be calculated (computed) as a negative area ratio per cell (Δarea/cell) by measuring, using LC/MS/MS, the absorbances of pyruvic acid, cystine, and serine in the medium after the culture of mesenchymal stem cells is started and the passaging is repeated a predetermined times, relative to the absorbances of a fresh medium containing no cells (i.e., the medium before the start of the culture), and then dividing the absorbances by the number of cells collected after the passaging is repeated the predetermined times.

The number, percentage, or the like of mesenchymal stem cells in a (cultured) cell population that are used in the evaluation method of the present invention can be obtained using a method known per se. Specifically, the number, percentage, or the like can be determined through flow cytometry or the like using a surface antigen (e.g., CD73, CD90, or CD105) of mesenchymal stem cells as an indicator.

The "step of evaluating the cells based on measurements of consumptions of pyruvic acid, cystine, and serine by the cells" of the present invention is a step of determining whether or not the cells fall within the consumption ranges of pyruvic acid, cystine, and serine serving as predetermined references (reference values) (or cutoff values).

Regarding the references above, when the consumptions of at least two of pyruvic acid, cystine, and serine and preferably the consumptions of at least two of these substances by one cell, or the consumptions of all of these substances and preferably the consumptions of all of these substances by one cell tend to increase (or acceleratingly increase), for example, over any period of the culture of mesenchymal stem cells (e.g., between the n^{th} and n+1^{th} passages, between the n^{th} and n+2^{th} passages, or between the n^{th} and n+3^{th} passages), the cells or cell population can be determined to have poor quality after the n^{th} passage and be unsuitable for, for example, medical applications (e.g., cell transplantation) and research applications.

In this specification, predetermined reference values (or cutoff values) of the consumptions of pyruvic acid, cystine, and serine are, for example, as follows.

The consumptions of pyruvic acid, cystine, and serine by the cells can be measured and calculated (computed) using, for example, the same method as that in Examples, which will be described later, and in this case, the predetermined reference values (cutoff values) therefor may be instantaneous consumptions, consumptions during a particular period (e.g., while the passaging is repeated 1, 2, or 3 times), or values obtained by comparing the consumptions before and after the passage (differences in the consumptions between before and after the passage) (e.g., division or subtraction of Δarea/cell before and after the passage). "Comparing the consumptions before and after the passage" may be as follows:
(i) comparing the consumptions at the time when the evaluation method of the present invention is conducted after the passaging is repeated, with the consumptions after the passaging is repeated predetermined reference times (e.g., 0, 1, 2, or 3 times, and preferably 3 times);
(ii) comparing the consumptions at the time when the evaluation method of the present invention is conducted after the n^{th} passage, with the consumptions after the n-1^{th} passage, where n-1 is the reference passage number; or
(iii) comparing the consumptions at the time when the evaluation method of the present invention is conducted after the n^{th} passage, with the consumptions after the n-2^{th} passage, where n-2 is the reference passage number.

In the present invention, "differences in the consumptions between before and after the passage" and "values obtained by comparing the consumptions before and after the passage" are interchangeably used, and there is no particular limitation on a method for calculating these values as long as the consumptions can be compared. For example, as described in (i) to (iii) above, values obtained by dividing the consumptions of pyruvic acid, cystine, and serine by the cells at the time when the evaluation method of the present invention is conducted after the passaging is repeated by the consumptions of pyruvic acid, cystine, and serine by the cells after the passaging is repeated reference times, or values obtained by subtracting the consumptions after the passaging is repeated reference times from the consumptions at the time when the evaluation method of the present invention is conducted after the passaging is repeated are used.

In this case, predetermined reference values (cutoff values) are values (after passage / before passage) that are obtained by dividing the consumptions of all of pyruvic acid, cystine, and serine after the passage by those before the passage, and preferably dividing the consumptions of all of pyruvic acid, cystine, and serine by one cell after the passage by those before the passage, and that indicate that the consumptions tend to increase after the passage compared with before the passage, and may be, for example, 1.1 times, 1.2 times, 1.3 times, 1.4 times, 1.5 times, 1.6 times, 1.7 times, 1.8 times, 1.9 times, 2.0 times, 2.1 times, 2.2 times, 2.3 times, 2.4 times, 2.5 times, 2.6 times, 2.7 times, 2.8 times, 2.9 times, 3.0 times, 3.1 times, 3.2 times, 3.3 times, 3.4 times, 3.5 times, 3.6 times, 3.7 times, 3.8 times, 3.9 times, 4.0 times, 4.1 times, 4.2 times, 4.3 times, 4.4 times, 4.5 times, 4.6 times, 4.7 times, 4.8 times, 4.9 times, 5.0 times, 5.1 times, 5.2 times, 5.3 times, 5.4 times, 5.5 times, 5.6 times, 5.7 times, 5.8 times, 5.9 times, 6.0 times, 6.1 times, 6.2 times, 6.3 times, 6.4 times, 6.5 times, 6.6 times, 6.7 times, 6.8 times, 6.9 times, 7.0 times, 7.1 times, 7.2 times, 7.3 times, 7.4 times, 7.5 times, 7.6 times, 7.7 times, 7.8 times, 7.9 times, or 8.0 times. When the reference values (or cutoff values) for the consumptions of all of pyruvic acid, cystine, and serine by mesenchymal stem cells or a mesenchymal stem cell population used in the evaluation method of the present invention reach or exceed the value above, the cells or cell population can be determined to have poor quality and be unsuitable for, for example, medical applications (e.g., cell transplantation) and research applications.

When the consumptions of pyruvic acid, cystine, and serine by the cells measured and calculated (computed) using the same method as that in Examples, which will be described later, predetermined reference values (cutoff values) therefor may be values obtained by comparing the consumptions before and after the passage (e.g., subtraction of Δarea/cell before and after the passage). In this case, predetermined reference values (cutoff values) are values (after passage - before passage) that are obtained by subtracting the consumptions of at least two of pyruvic acid, cystine, and serine before the passage from those after the passage, and preferably subtracting the consumptions of at least two of pyruvic acid, cystine, and serine by one cell before the passage from those after the passage, or subtracting the consumptions of all of these substances before the passage from those after the passage, and preferably subtracting the consumptions of all of these substances by one cell before the passage from those after the passage, and that indicate that the consumptions tend to increase after the passage compared with before the passage, and may be, for example, -1.0, -1.1, -1.2, -1.3, -1.4, -1.5, -1.6, -1.7, -1.8, -1.9, -2.0, -2.1, -2.2, -2.3, -2.4, -2.5, -2.6, -2.7, -2.8, -2.9, -3.0, -3.1, -3.2, -3.3, -3.4, -3.5, -3.6, -3.7, -3.8, -3.9, -4.0, -4.1, -4.2, -4.3, -4.4, -4.5, -4.6, -4.7, -4.8, -4.9, -5.0, -5.1, -5.2, -5.3, -5.4, -5.5, -5.6, -5.7, -5.8, -5.9, -6.0, -6.1, -6.2, -6.3, -6.4, -6.5, -6.6, -6.7, -6.8, -6.9, -7.0, -7.1, -7.2, -7.3, -7.4, -7.5, -7.6, -7.7, -7.8, -7.9, -8.0, -8.1, -8.2, -8.3, -8.4, -8.5, -8.6, -8.7, -8.8, -8.9, -9.0, -10, -15, -18, or -20. When the reference values (or cutoff values) for the consumptions of at least two or all of pyruvic acid, cystine, and serine by mesenchymal stem cells or a mesenchymal stem cell population used in the evaluation method of the present invention reach or fall below the value above, the cells or cell population can be determined to have poor quality and be unsuitable for, for example, medical applications (e.g., cell transplantation) and research applications.

Regarding the above-described reference values (cutoff values), when values obtained by comparing the consumptions of all of pyruvic acid, cystine, and serine before and after the passage are greater than or equal to (in the case of "after passage / before passage") or smaller than or equal to (in the case of "after passage - before passage") the above-described reference value (cutoff value) two or more times in a row (e.g., the evaluation method of the present invention is conducted after the n^{th} passage (the reference passage number is n-1) and the n+1^{th} passage (the reference passage number is n)), the cells or cell population may be determined to have poor quality and be unsuitable for, for example, medical applications (e.g., cell transplantation) and research applications.

Although there is no particular limitation on a source species of mesenchymal stem cells used in the evaluation method of the present invention, examples of the source species include rodents such as a rat, a mouse, a hamster, and a guinea pig, lagomorphs such as rabbit, ungulates such as a pig, a cow, a goat, and a sheep, carnivores such as a dog and a cat, and primates such as a human, a monkey, a rhesus monkey, a marmoset, an orangutan, and a chimpanzee.

All the contents described in "1. Method for Evaluating Quality of Mesenchymal Stem Cells According to the Present Invention (Mitochondrial Size, etc.)", "2. Method for Producing Mesenchymal Stem Cells According to the Present Invention (Mitochondrial Size, etc.)", and "3. Method for Producing Mesenchymal Cells According to the Present Invention (Mitochondrial Size, etc.)" are applied to the matters required to conduct the method for evaluating a medium for mesenchymal stem cells (consumptions of pyruvic acid, cystine, and serine, etc.) according to the present invention other than those described above.

### 7. Method for Producing Mesenchymal Stem Cells According to the Present Invention (Consumptions of Pyruvic Acid, Cystine, and Serine, etc.)

The present invention relates to a method for producing mesenchymal stem cells evaluated for quality, the method including a step of evaluating the cells (a step of evaluating quality) based on measurements of consumptions of pyruvic acid, cystine, and serine by the cells, and also relates to mesenchymal stem cells obtained using this production method. More specifically, the method for producing mesenchymal stem cells according to the present invention includes: (A) a step of preparing mesenchymal stem cells to be subjected to expansion culture; and (B) a step of conducting expansion culture of the prepared cells, and the step of evaluating quality may be conducted before the step (A), during the step (A), after the step (A), during the step (B), or after the step (B). The step of evaluating quality may be conducted once in a particular period or a plurality of times. In an aspect of the present invention, the present invention includes: (1) a step of preparing mesenchymal stem cells evaluated for quality; and (2) a step of culturing the mesenchymal stem cells.

All the contents described in "1. Method for Evaluating Quality of Mesenchymal Stem Cells According to the Present Invention (Mitochondrial Size, etc.)", "2. Method for Producing Mesenchymal Stem Cells According to the Present Invention (Mitochondrial Size, etc.)", "3. Method for Producing Mesenchymal Cells According to the Present Invention (Mitochondrial Size, etc.)", and "6. Method for Evaluating Quality of Mesenchymal Stem Cells According to the Present Invention (Consumptions of Pyruvic Acid, Cystine, and Serine, etc.)" are applied to the matters required to conduct the method for producing mesenchymal stem cells (consumptions of pyruvic acid, cystine, and serine, etc.) according to the present invention other than those described above.

### 8. Method for Producing Mesenchymal Cells According to the Present Invention (Consumptions of Pyruvic Acid, Cystine, and Serine, etc.)

The present invention relates to a method for producing mesenchymal cells (differentiated cells), the method including a step of inducing differentiation of mesenchymal stem cells obtained using the method for producing mesenchymal stem cells (consumptions of pyruvic acid, cystine, and serine, etc.) according to the present invention, and also relates to mesenchymal cells obtained using this production method. More specifically, the present invention includes: (1) a step of preparing mesenchymal stem cells obtained using the method for producing mesenchymal stem cells according to the present invention; and (2) a step of inducing differentiation into the mesenchymal cells. Examples of such mesenchymal cells include osteocytes, chondrocytes, and adipocytes. It is preferable to conduct all the steps of the method for producing mesenchymal cells according to the present invention under the feeder-free and xeno-free conditions.

All the contents described in "1. Method for Evaluating Quality of Mesenchymal Stem Cells According to the Present Invention (Mitochondrial Size, etc.)", "2. Method for Producing Mesenchymal Stem Cells According to the Present Invention (Mitochondrial Size, etc.)", "3. Method for Producing Mesenchymal Cells According to the Present Invention (Mitochondrial Size, etc.)", "6. Method for Evaluating Quality of Mesenchymal Stem Cells According to the Present Invention (Consumptions of Pyruvic Acid, Cystine, and Serine, etc.)", and "7. Method for Producing Mesenchymal Stem Cells According to the Present Invention (Consumptions of Pyruvic Acid, Cystine, and Serine, etc.)" are applied to the matters required to conduct the method for producing mesenchymal stem cells (consumptions of pyruvic acid, cystine, and serine, etc.) according to the present invention other than those described above.

### 9. Cell Transplantation Therapy Agent (Consumptions of Pyruvic Acid, Cystine, and Serine, etc.)

The mesenchymal stem cells obtained using the method for producing mesenchymal stem cells (consumptions of pyruvic acid, cystine, and serine, etc.) according to the present invention and the mesenchymal cells (differentiated cells) obtained using the method for producing mesenchymal cells (consumptions of pyruvic acid, cystine, and serine, etc.) according to the present invention (these cells may be collectively referred to as "mesenchymal cells of the present invention" hereinafter) can have excellent repairing ability when transplanted to a defect site of the bone, cartilage, muscle, or the like, compared with cells obtained using conventional methods. Accordingly, the mesenchymal cells of the present invention can be favorably used in cell transplantation therapy. Therefore, the present invention relates to a cell transplantation therapy agent containing the mesenchymal cells of the present invention. In addition, the present invention also encompasses a method for treating an injury (including defect) of a tissue (e.g., a bone tissue, a cartilage tissue, an adipose tissue, and a muscle tissue (e.g., skeletal muscle tissue)) or a disease, the method including administering or transplanting an effective amount of the mesenchymal cells of the present invention to a mammal (e.g., a human, a mouse, a rat, a monkey, a cow, a horse, a pig, or a dog) to be treated. Also, the "treatment of an injury of a tissue" encompasses regeneration of an injured tissue.

All the contents described in "1. Method for Evaluating Quality of Mesenchymal Stem Cells According to the Present Invention (Mitochondrial Size, etc.)", "2. Method for Producing Mesenchymal Stem Cells According to the Present Invention (Mitochondrial Size, etc.)", "3. Method for Producing Mesenchymal Cells According to the Present Invention (Mitochondrial Size, etc.)", "6. Method for Evaluating Quality of Mesenchymal Stem Cells According to the Present Invention (Consumptions of Pyruvic Acid, Cystine, and Serine, etc.)", "7. Method for Producing Mesenchymal Stem Cells According to the Present Invention (Consumptions of Pyruvic Acid, Cystine, and Serine, etc.)", and "8. Method for Producing Mesenchymal Cells According to the Present Invention (Consumptions of Pyruvic Acid, Cystine, and Serine, etc.)" are applied to the matters required to conduct the method for producing mesenchymal stem cells (consumptions of pyruvic acid, cystine, and serine, etc.) according to the present invention other than those described above.

### 10. Method for Evaluating Medium for Mesenchymal Stem Cells According to the Present Invention (Consumptions of Pyruvic Acid, Cystine, and Serine, etc.)

The present invention relates to a method for evaluating a medium for mesenchymal stem cells, the method including a step of evaluating the medium based on measurements of consumptions of pyruvic acid, cystine, and serine by the cells.

More specifically, an aspect of the present invention relates to a method for evaluating a medium for mesenchymal stem cells (evaluation method 1' of the present invention), the method including:
(1) a step of culturing mesenchymal stem cells in a medium to be evaluated;
(2) a step of measuring consumptions of pyruvic acid, cystine, and serine by the cultured mesenchymal stem cells; and
(3) a step of comparing the measured consumptions of pyruvic acid, cystine, and serine by the cells with predetermined reference values.

Specific examples of the consumptions of pyruvic acid, cystine, and serine include those described in "6. Method for Evaluating Quality of Mesenchymal Stem Cells According to the Present Invention (Consumptions of Pyruvic Acid, Cystine, and Serine, etc.)" above. When the consumptions are measured and calculated (computed) using, for example, the same method as that in Examples, which will be described later, predetermined reference values (cutoff values) therefor may be instantaneous consumptions, consumptions during a particular period (e.g., while the passaging is repeated 1, 2, or 3 times), or values obtained by comparing the consumptions before and after the passage (e.g., division or subtraction of Δarea/cell before and after the passage). When the consumptions before and after the passage are compared, predetermined reference values (cutoff values) are values (after passage / before passage) that are obtained by dividing the consumptions of all of pyruvic acid, cystine, and serine after the passage by those before the passage, and preferably dividing the consumptions of all of pyruvic acid, cystine, and serine by one cell after the passage by those before the passage, and that indicate that the consumptions tend to increase after the passage compared with before the passage, and may be, for example, 1.1 times, 1.2 times, 1.3 times, 1.4 times, 1.5 times, 1.6 times, 1.7 times, 1.8 times, 1.9 times, 2.0 times, 2.1 times, 2.2 times, 2.3 times, 2.4 times, 2.5 times, 2.6 times, 2.7 times, 2.8 times, 2.9 times, 3.0 times, 3.1 times, 3.2 times, 3.3 times, 3.4 times, 3.5 times, 3.6 times, 3.7 times, 3.8 times, 3.9 times, 4.0 times, 4.1 times, 4.2 times, 4.3 times, 4.4 times, 4.5 times, 4.6 times, 4.7 times, 4.8 times, 4.9 times, 5.0 times, 5.1 times, 5.2 times, 5.3 times, 5.4 times, 5.5 times, 5.6 times, 5.7 times, 5.8 times, 5.9 times, 6.0 times, 6.1 times, 6.2 times, 6.3 times, 6.4 times, 6.5 times, 6.6 times, 6.7 times, 6.8 times, 6.9 times, 7.0 times, 7.1 times, 7.2 times, 7.3 times, 7.4 times, 7.5 times, 7.6 times, 7.7 times, 7.8 times, 7.9 times, or 8.0 times. When the reference values (or cutoff values) for the consumptions of all of pyruvic acid, cystine, and serin by the mesenchymal stem cells or the mesenchymal stem cell population reach or exceed the value above, the medium can be determined to be unsuitable as a medium for mesenchymal stem cells.

When the consumptions of pyruvic acid, cystine, and serine by the cells are measured and calculated (computed) using the same method as that in Examples, which will be described later, predetermined reference values (cutoff values) therefor may be values obtained by comparing the consumptions before and after the passage (e.g., subtraction of Δarea/cell before and after the passage). In this case, predetermined reference values (cutoff values) are values (after passage - before passage) that are obtained by subtracting the consumptions of at least two of pyruvic acid, cystine, and serine before the passage from those after the passage, and preferably subtracting the consumptions of at least two of pyruvic acid, cystine, and serine by one cell before those after the passage, or subtracting the consumptions of all of these substances before the passage from those after the passage, and preferably subtracting the consumptions of all of these substances by one cell before the passage from those after the passage, and that indicate that the consumptions tend to increase after the passage compared with before the passage, and may be, for example, -1.0, -1.1, -1.2, -1.3, -1.4, -1.5, -1.6, -1.7, -1.8, -1.9, -2.0, -2.1, -2.2, -2.3, -2.4, -2.5, -2.6, -2.7, -2.8, -2.9, -3.0, -3.1, -3.2, -3.3, -3.4, -3.5, -3.6, -3.7, -3.8, -3.9, -4.0, -4.1, -4.2, -4.3, -4.4, -4.5, -4.6, -4.7, -4.8, -4.9, -5.0, -5.1, -5.2, -5.3, -5.4, -5.5, -5.6, -5.7, -5.8, -5.9, -6.0, -6.1, -6.2, -6.3, -6.4, -6.5, -6.6, -6.7, -6.8, -6.9, -7.0, -7.1, -7.2, -7.3, -7.4, -7.5, -7.6, -7.7, -7.8, -7.9, -8.0, -8.1, -8.2, -8.3, -8.4, -8.5, -8.6, -8.7, -8.8, -8.9, -9.0, -10, -15, -18, or -20. When the reference values (or cutoff values) for the consumptions of at least two or all of pyruvic acid, cystine, and serin by the mesenchymal stem cells or the mesenchymal stem cell population reach or fall below the value above, the medium can be determined to be unsuitable as a medium for mesenchymal stem cells.

All the contents described in "1. Method for Evaluating Quality of Mesenchymal Stem Cells According to the Present Invention (Mitochondrial Size, etc.)", "2. Method for Producing Mesenchymal Stem Cells According to the Present Invention (Mitochondrial Size, etc.)", "3. Method for Producing Mesenchymal Cells According to the Present Invention (Mitochondrial Size, etc.)", "6. Method for Evaluating Quality of Mesenchymal Stem Cells According to the Present Invention (Consumptions of Pyruvic Acid, Cystine, and Serine, etc.)", "7. Method for Producing Mesenchymal Stem Cells According to the Present Invention (Consumptions of Pyruvic Acid, Cystine, and Serine, etc.)", and "8. Method for Producing Mesenchymal Cells According to the Present Invention (Consumptions of Pyruvic Acid, Cystine, and Serine, etc.)" are applied to the matters required to conduct the method for evaluating a medium for mesenchymal stem cells (consumptions of pyruvic acid, cystine, and serine, etc.) according to the present invention other than those described above.

Hereinafter, the present invention will be described more specifically by way of examples, but the present invention is not limited to these examples by any means.

### Examples

### <Materials and Methods>

Materials and the like used in the culture of mesenchymal stem cells are shown in Table 1 below.

**[Table 1]**

| Material name | Manufacturer | Model number | Details | Notes |
|---|---|---|---|---|
| MSC, derived from fat | LONZA | PT-5006 | 42-year-old woman | Cell culture |
| MSC, derived from fat | LONZA | PT-5006 | 58-year-old man | Cell culture |
| CiMS-BM | NIPRO | 87-070 | | Cell culture |
| CiMS-sAF | NIPRO | 87-072 | | Cell culture |
| Cellartis (registered trademark) MSC Xeno-Free Culture Medium | TaKaRa | Y50200 | | Cell culture |
| Vitronectin (VTN-N), 1 mL | Thermo Fisher | A14700 | | Cell culture |
| Stempro (tredemark) Ezpassage (trademark) Disposable stem cell passaging Tool | Thermo Fisher | 23181010 | | Cell culture |
| KBM ADSC-4 | KOHJIN BIO | 16030044 | | Cell culture |
| Falcon (registered trademark) 6-well Flat Bottom Multiwell Cell Culture Plate, with Lid | BD (Falcon) | 353046 | | Cell culture |
| CellBIND (registered trademark) 6-well Clear Multiple Well Plates, Flat Bottom, with Lid, Sterile | Corning | 3335 | | Cell culture |

Materials (FACS antibodies, etc.) and the like used in flow cytometry and mitochondrial membrane charge detection are shown in Table 2.

**[Table 2]**

| Product name | Manufacturer | Cat No. |
|---|---|---|
| BV421 Mouse Anti-Human CD90 | BD Horizon | 562256 |
| APC Mouse Anti-Human CD73 | BD Pharmingen | 560847 |
| 7AAD | BD Pharmingen | 559925 |
| AIE Mitochondria Red | AlEgen Biotech | BPM670 |
| DPBS, no calcium, no magnesium | Thermo Fisher | 14190144 |
| Donated blood albumin 25% IV Injection 12.5 g/50 mL "Benesis" | Japan Blood Products Organization | A484PX |

Materials used in viability test performed using an animal with the MSC passage number being varied are shown in Table 3.

**[Table 3]**

| Material name | Manufacturer | Model number |
|---|---|---|
| Matrigel, basal membrane matrix | Corning | 356231 |
| NOD/Shi-scid, IL-2RγKO Jic | In-Vivo Science Inc. | |

### Example 1: Test for Culture of Mesenchymal Stem Cells

### (Activation)

### (i) On Day before Activation

On the day before activation of frozen cells, in accordance with the manufacturer's protocol, a 6-well plate was coated with VTN-N, was left to stand at room temperature for 1 hour, and was then stored in the refrigerator.

### (ii) On Day of Activation

A necessary amount of medium and the VTN-N-coated plate were brought to room temperature.
1. The cell vial was removed from the storage container and quickly thawed in a water bath at 37°C.
2. 5 ml of a proliferation medium was used per 0.5 ml of the thawed cell solution, and the resulting cell suspension was transferred to a 14-ml tube.
3. After the suspension was centrifuged at 1500 rpm for 5 min at R.T, the supernatant was removed using an aspirator, the cells were loosened through tapping, and 2 ml of the medium was added.
4. The number of viable cells was counted. The viable cells were seeded at a seeding concentration of 3×10⁴ viable cells/2 ml/well.
5. The culture was started in an incubator at 37°C and 5% CO₂ (Day 0).
6. The day when the cells were seeded was defined as day 0, and a microscopic examination and a passage were conducted on day 4. The medium was not replaced during this period.

### (Passage)

### (i) On Day before Activation

On the day before activation of frozen cells, in accordance with the manufacturer's protocol, a 6-well plate was coated with VTN-N, was left to stand at room temperature for 1 hour, and was then stored in the refrigerator.

### (ii) On Day of Activation

A necessary amount of medium and the VTN-N-coated plate were brought to room temperature.
1. The medium was removed using an aspirator.
2. 2 ml/well of PBS was added, and after the plate was lightly shaken, PBS was removed using an aspirator.
3. 0.3 to 0.5 ml/well of a cell detachment agent was added and incubated at 37°C for 3 to 5 minutes.
4. 2 ml/well of the medium was added to 3 above, and the resulting suspension was transferred to a conical tube and centrifuged at 1500 rpm for 5 min at R.T.
5. The supernatant obtained in 4 above was removed using an aspirator, the cells were loosened through tapping, and 2 ml of the medium was added.
6. The number of viable cells was counted. The viable cells were seeded at a seeding concentration of 3×10⁴ viable cells/2 ml/well.
7. The culture was started in an incubator at 37°C and 5% CO₂ (Day 0).
8. The day when the cells were seeded was defined as day 0, and a passage was conducted on day 4. The medium was not replaced until the passage.

### (Proliferation Ratio and Proliferation Curve)

The proliferation ratios and the proliferation curves for MSC-1 passaged as described above using 4 types of media (CiMS_MSC-1, StemPro_MSC-1, ADSC-4_MSC-1, and Cellartis_MSC-1) were determined (FIGS. 1 and 2).

### (Results)

It was found from the results of the test above that the number of passages that caused a decrease in the proliferation ratio varied depending on the type of medium in which MSCs were cultured (FIGS. 1 and 2).

### Example 2: Detection of Mitochondrial Membrane Potential

5 mM AIE RED in DMSO was prepared (stock solution preparation).
1. The AIE RED solution in an amount of 1/1000 of the medium was added to mesenchymal stem cells in 1 well of a 6-well plate on day 4 of the culture, mixed thoroughly, and incubated in an incubator at 37°C and 5% CO₂ for 45 minutes.
2. The medium was removed using an aspirator after 45 minutes. 2 ml/well of PBS was added, and the plate was lightly shaken. Then, 2 ml of the medium was added again, and observation was conducted under a microscope.

### (Results)

It was found that, at Passage 3 (P3), which is a small passage number, each mitochondrion was short, whereas at Passage 8 (P8), which is a larger passage number, mitochondria fused to each other, resulting in an increase in length (FIG. 3). It could be understood from these results that there was a relationship between the mitochondrial size and the passage number.

### Example 3: Mitochondrial Charge Analysis

5 mM AIE RED in DMSO was prepared (stock solution preparation).
1. The AIE RED solution in an amount of 1/1000 of the medium was added to mesenchymal stem cells in 1 well of a 6-well plate on day 4 of the culture, mixed thoroughly, and incubated in an incubator at 37°C and 5% CO₂ for 45 minutes.
2. 2 ml/well of PBS was added after 45 minutes, and after the plate was lightly shaken, PBS was removed using an aspirator.
3. 0.3 to 0.5 ml/well of a cell detachment agent was added and incubated at 37°C for 3 to 5 minutes.
4. 2 ml/well of the medium was added to 3 above, and the resulting suspension was transferred to a conical tube and centrifuged at 1500 rpm for 5 min at R.T.
5. The supernatant obtained in 4 above was removed using an aspirator, the cells were loosened through tapping, and 0.5 ml of a 2% HAS solution was added.
6. A measurement was conducted using flow cytometry (BD FACSAriaII Cell Sorter).

### (Results)

Mitochondrial changes depending on the passage number of MSC-1 CiMS are shown with the vertical axis illustrating the mitochondrial charge and the horizontal axis illustrating the cell size (FIGS. 4 and 6). In particular, when the region of a mitochondrial charge of 5.0 K or more and the cell size of 120 K or more (in this region, CiMS shows a mitochondrial charge of 5.0 K and a cell size of 120 K when FACS is set to "PE: voltage 290" and the cell population at P3 is used as a reference) was examined, the mitochondrial size started to change at approximately P6 (10.4% (FIG. 4), 7.7% (FIG. 6)), and this result tended to correspond to the proliferation ratio of MSC-1 CiMS in FIG. 1. Also, regarding mitochondrial changes depending on the passage number of MSC-1 Cellartis, particularly in the region of a mitochondrial charge of 2.5 K or more and the cell size of 90 K or more (in this region, Cellartis shows a mitochondrial charge of 2.5 K and a cell size of 90 K when FACS is set to "PE: voltage 290" and the cell population at P3 is used as a reference), the mitochondrial size started to change at approximately P15 (22.6% (FIG. 5)) or P11 (11.0% (FIG. 7)), and this result tended to correspond to the proliferation ratio of MSC-1 Cellartis in FIG. 2. It was found from these results that the quality of mesenchymal stem cells can be evaluated using, as an indicator, the mitochondrial charge (size) or the mitochondrial charge (size) in cells with a size larger than or equal to a particular size.

### Example 4: Membrane Potential Analysis

5 mM AIE RED in DMSO was prepared (stock solution preparation).
1. The medium of mesenchymal stem cells in 1 well of a 6-well plate was removed using an aspirator on day 4 of the culture, and the cells were washed with PBS once.
2. 0.3 to 0.5 ml/well of a cell detachment agent was added and incubated at 37°C for 3 to 5 minutes.
3. 2 ml/well of the medium was added to 2 above, and the resulting suspension was transferred to a conical tube and centrifuged at 1500 rpm for 5 min at R.T.
4. The supernatant obtained in 3 above was removed using an aspirator, the cells were loosened through tapping, and 0.2 ml of a 2% HAS solution was added.
5. After appropriate dilution, the number of cells was counted and adjusted to 2.5×10⁵ cells/0.1 ml. Then, 1 µl of CD73, 1 µl of CD90, and 1 µl of 7AAD were added and reacted at room temperature for 20 minutes.
6. After 20 minutes, 1 ml of 2% HAS solution was added, the resulting suspension was centrifuged at 3000 rpm for 2 min at R.T, and the supernatant was removed.
7. The cells were tapped, 1 ml of 2% HAS solution was added again, the resulting suspension was centrifuged at 3000 rpm for 2 min at R.T, and the supernatant was removed.
8. The cells were tapped, 0.3 ml of 2% HAS solution was added again, and the cells were passed through a strainer for FACS.
9. A measurement was conducted using flow cytometry (BD FACSAriaII Cell Sorter).

### (Results)

Tables 4 and 5 show the results of the membrane potential analysis.

**[Table 4]**

| Passage number | Number of cells | % of cells with size of 100 K or less | Mean of mitochondria in cells with size of 100 K or less | % of cells with size of 100 K or more | Mean of mitochondria in cells with size of 100 K or more |
|---|---|---|---|---|---|
| 3 | 10193 | 77.5 | 1249 | 22.5 | 3345 |
| 6 | 10028 | 58.7 | 1104 | 41.3 | 3568 |
| 7 | 10241 | 63.9 | 984 | 36.1 | 3221 |
| 8 | 10224 | 60.8 | 1053 | 39.2 | 2951 |
| 9 | 10665 | 34.7 | 1290 | 65.3 | 4614 |
| 10 | 10407 | 44.4 | 1355 | 55.6 | 4924 |

**[Table 5]**

| Passage number | Number of cells | % of cells with size of 100 K or less | Mean of mitochondria in cells with size of 100 K or less | % of cells with size of 100 K or more | Mean of mitochondria in cells with size of 100 K or more |
|---|---|---|---|---|---|
| P3 | 10048 | 96.4 | 844 | 3.55 | 2777 |
| P6 | 9968 | 88.7 | 1034 | 11.3 | 2549 |
| P7 | 10087 | 87.7 | 810 | 12.3 | 2346 |
| P8 | 10090 | 73.7 | 901 | 26.3 | 1804 |
| P9 | 10085 | 84.6 | 727 | 15.4 | 2206 |
| P15 | 10303 | 63.4 | 1093 | 36.6 | 3345 |
| P18 | 10444 | 53.2 | 1034 | 46.8 | 2500 |

It was found from Tables 4 and 5 that the cells with a size of 100 K or less retained substantially the same brightness as that of Passage 3 (P3) even when the passaging was repeated, whereas the brightness of the cells with a size of 100 K or more (the cells with a diameter of about 30 µm or more) tended to increase after the passaging was repeated (FIGS. 8 and 9). Accordingly, it was found that the brightness (fluorescence intensity) of mitochondria obtained through flow cytometry measurement, particularly the brightness (average value) of mitochondria in the cells with a size of 100 K or more, can be used as an indicator for evaluation of the quality of mesenchymal stem cells (FIGS. 8 and 9).

FIGS. 10 and 11 show the results of examination of the CV values (the Coefficient of Variation is a normalized Standard Deviation), namely StdDev (standard deviation, variation) / Mean (mean for standard distribution), obtained from the measurement results of flow cytometry. It is understood from these diagrams that using "CV value ≤ 50.0" as an indicator is effective in evaluation of the quality of mesenchymal stem cells.

### Example 5: Viability Test Performed Using Animal with MSC Passage Number Being Varied

### (Object)

When cells at Passage 3 and Passage 8 of fat-derived MSCs in the CiMS medium are transplanted to mice, and cells at Passage 4, Passage 7, and Passage 10 in the Cellartis medium (TAKARA) are transplanted to mice, it is confirmed whether or not the transplanted cells are viable as MSCs.

### (Mouse Subcutaneous Transplantation - Cell Collection - Reculture - FACS Analysis)

After the reactivation, MSC-1 (fat-derived MSC cells) CiMS (Nipro)/VTN (Thermo) or Cellartis (TAKARA) were cultured for 4 days to prepare 2.5×10⁶ cells/1.5 ml tube, were pelleted, and were then placed on ice until use.
1. NOD mice (In-Vivo Science Inc., n=3) were anesthetized.
2. The cells were mixed with Matrigel (Corning Incorporated) and were subcutaneously transplanted at 20×10⁵ cells/mouse.
3. After 2 weeks (the passage was conducted every 4 days, resulting in 3 passages in 2 weeks, that is, Passage 8 corresponded to Passage 11, which was used as a reference), the cells were collected from the mice and were immersed in TrypLE select × 10 (Thermo Fisher Scientific) for about 30 minutes together with the Matrigel.
4. The Matrigel was finely cut using scissors and was seeded on a dish. At this time, cover glass or the like was used to prevent the Matrigel from floating.
5. After the Matrigel was cultured in Cellartis or CiMS (the same medium before the transplantation) for about 1 week, CD90 and CD73 were analyzed using FACS (BD FACSAriaII Cell Sorter).

### (Results)

When CiMS (Nipro) was used, as a result of the reculture after the transplantation to the mice, the transplanted MSCs (CD90-CD73 double positive cells) made up about 54% at Passage 3 (P3) but about 0.5% at Passage (P8) (FIG. 14). When Cellartis (TAKARA) was used, as a result of the reculture after the transplantation to the mice, the transplanted MSCs (CD90-CD73 double positive cells) made up about 75% at Passage 4 (P4), about 48% at Passage 7 (P7), and about 10% at Passage 10 (P10) (FIG. 15). It was understood from these results that, when the transplantation was conducted after the passaging was repeated appropriate times, more transplanted cells were viable as MSCs. Determination of whether the passage number is appropriate significantly corresponded to the results of the evaluation of the quality of MSCs using the mitochondrial size as an indicator, which has been confirmed in Examples 1 to 3, and it is understood from these results as well that the mitochondrial size is an excellent indicator for the evaluation of the quality of MSCs.

### Example 6: Examination of Consumptions of Pyruvic Acid, Cystine, and Serine by One Cell

The reactivation and passages of mesenchymal stem cells were conducted using the same materials and methods as those in Example 1, a liquid chromatograph/tandem mass spectrometer (LC/MS/MS) (LCMS-8050 and LC/MS/MS method package cell culture profiling (SHIMADZU CORPORATION)) was used at every passages (the third to tenth passages (CiMS) and the third to thirteenth passages (Cellartis)) to measure the absorbances of pyruvic acid, cystine, and serine in the medium at the passages above after the culture of the mesenchymal stem cells was started, relative to the absorbances of a fresh medium containing no cells (i.e., the medium before the start of the culture), and then the measured absorbances were divided by the number of cells collected at the passages to calculate (compute) a negative area ratio per cell (Δarea/cell).

### (Results)

Tables 6, 7 and 8 (CiMS) and Tables 9, 10, and 11 (Cellartis) below show the consumptions of pyruvic acid, cystine, and serine by one cell at every passage, differences in the consumptions between before and after the passage (after passage / before passage, and after passage - before passage), and differences in consumptions with respect to the passage P3 (after passage / P3, and after passage - P3).

**[Table 6]**

| Passage number | Number of cells | Δarea/cell (Pyruvic acid, cystine, serine) |
|---|---|---|
| P3 | 5.6×10⁵ | -0.48, -3.6, -1.8 |
| P4 | 2.7×10⁵ | -1.1, -5.3, -2.5 |
| P5 | 2.4×10⁵ | -1.3, -5.1, -2.8 |
| P6 | 1.7×10⁵ | -1.7, -5.8, -2.8 |
| P7 | 1.3×10⁵ | -1.8, -3.9, -2.7 |
| P8 | 1.4×10⁵ | -2.6, -9.3, -4.0 |
| P9 | 9.5×10⁴ | -3.4, -15.9, -5.3 |
| P10 | 4.5×10⁴ | -8.7, -21.2, -8.7 |

**[Table 7]**

| After passage / before passage | (Pyruvic acid, cystine, serine) | After passage - before passage | (Pyruvic acid, cystine, serine) |
|---|---|---|---|
| P4/P3 | 2.2, 1.5, 1.4 | P4-P3 | -0.58, -1.7, -0.71 |
| P5/P4 | 1.2, 0.96, 1.1 | P5-P4 | -0.25, 0.23, -0.33 |
| P6/P5 | 1.3, 1.1, 0.99 | P6-P5 | -0.43, -0.74, 0.019 |
| P7/P6 | 1.0, 0.67, 0.95 | P7-P6 | -0.038, 1.9, 0.13 |
| P8/P7 | 1.5, 2.4, 1.5 | P8-P7 | -0.81, -5.4, -1.3 |
| P9/P8 | 1.3, 1.7, 1.3 | P9-P8 | -0.86, -6.7, -1.3 |
| P10/P9 | 2.5, 1.3, 1.6 | P10-P9 | -5.3, -5.3, -3.4 |

**[Table 8]**

| After passage / P3 | (Pyruvic acid, cystine, serine) | After passage - P3 | (Pyruvic acid, cystine, serine) |
|---|---|---|---|
| P4/P3 | 2.2, 1.5, 1.4 | P4-P3 | -0.58, -1.7, -0.71 |
| P5/P3 | 2.7, 1.4, 1.6 | P5-P3 | -0.83, -1.5, -1.0 |
| P6/P3 | 3.6, 1.6, 1.6 | P6-P3 | -1.3, -2.2, -1.0 |
| P7/P3 | 3.7, 1.1, 1.5 | P7-P3 | -1.3, -0.27, -0.90 |
| P8/P3 | 5.4, 2.6, 2.3 | P8-P3 | -2.1, -5.7, -2.2 |
| P9/P3 | 7.2, 4.4, 3.0 | P9-P3 | -3.0, -12, -3.6 |
| P10/P3 | 18, 5.9, 5.0 | P10-P3 | -8.3, -18, -7.0 |

**[Table 9]**

| Passage number | Number of cells | Δarea/cell (Pyruvic acid, cystine, serine) |
|---|---|---|
| P3 | 4.3×10⁵ | -0.4, -2.8, -3.3 |
| P4 | 5.2×10⁵ | -0.4, -2.4, -2.3 |
| P5 | 2.9×10⁵ | -0.9, -3.7, -3.6 |
| P6 | 4.5×10⁵ | -0.5, -3.5, -2.8 |
| P7 | 3.5×10⁵ | -0.7. -3.3, -2.9 |
| P8 | 4.0×10⁵ | -0.5, -3.2, -3.0 |
| P9 | 4.2×10⁵ | -0.4, -3.5, -3.1 |
| P10 | 4.4×10⁵ | -0.4, -3.2, -3.3 |
| P11 | 3.7×10⁵ | -0.5, -2.1, -2.9 |
| P12 | 2.0×10⁵ | -1.1, -4.8, -4.6 |
| P13 | 1.1×10⁵ | -2.0, -7.4, -7.8 |

**[Table 10]**

| After passage / before passage | (Pyruvic acid, cystine, serine) | After passage - before passage | (Pyruvic acid, cystine, serine) |
|---|---|---|---|
| P4/P3 | 1.1, 0.86, 0.68 | P4-P3 | -0.032, 0.40, 1.1 |
| P5/P4 | 2.1, 1.6, 1.6 | P5-P4 | -0.47, -1.3, -1.3 |
| P6/P5 | 0.56, 0.96, 0.78 | P6-P5 | 0.39, 0.16, 0.78 |
| P7/P6 | 1.4, 0.93, 1.0 | P7-P6 | -0.18, 0.25, -0.13 |
| P8/P7 | 0.70, 0.97, 1.0 | P8-P7 | 0.21, 0.10, -0.092 |
| P9/P8 | 0.89, 1.1, 1.0 | P9-P8 | 0.053, -0.34, -0.054 |
| P10/P9 | 1.0, 0.92, 1.1 | P10-P9 | 0.00, 0.30, -0.20 |
| P11/P10 | 1.1, 0.66, 0.88 | P11-P10 | -0.043, 1.1, 0.38 |
| P12/P11 | 2.4, 2.2, 1.6 | P12-P11 | -0.64, -2.6, -1.7 |
| P13/P12 | 1.7, 1.6, 1.7 | P13-P12 | -0.81, -2.6, -3.2 |

**[Table 11]**

| After passage / P3 | (Pyruvic acid, cystine, serine) | After passage - P3 | (Pyruvic acid, cystine, serine) |
|---|---|---|---|
| P4/P3 | 1.1, 0.86, 0.68 | P4-P3 | -0.032, 0.40, 1.1 |
| P5/P3 | 2.3, 1.3, 1.1 | P5-P3 | -0.50, -0.93, -0.24 |
| P6/P3 | 1.3, 1.3, 0.84 | P6-P3 | -0.11, -0.78, 0.54 |
| P7/P3 | 1.7, 1.2, 0.88 | P7-P3 | -0.29, -0.52, 0.41 |
| P8/P3 | 1.2, 1.2, 0.91 | P8-P3 | -0.082, -0.42, 0.32 |
| P9/P3 | 1.1, 1.3, 0.92 | P9-P3 | -0.029, -0.76, 0.26 |
| P10/P3 | 1.1, 1.2, 0.98 | P10-P3 | -0.030, -0.47, 0.066 |
| P11/P3 | 1.2, 0.77, 0.87 | P11-P3 | -0.072, 0.64, 0.45 |
| P12/P3 | 2.8, 1.7, 1.4 | P12-P3 | -0.71, -2.0, -1.2 |
| P13/P3 | 4.9, 2.7, 2.3 | P13-P3 | -1.5, -4.6, -4.5 |

When CiMS was used as the medium for mesenchymal stem cells, the consumptions of pyruvic acid, cystine, and serine all increased after the seventh passage (P7), and the consumptions of these three substances all increased at the eighth passage (P8) to the tenth passage (P10) as well (FIG. 16). When Cellartis was used as the medium, the consumptions of pyruvic acid, cystine, and serine all increased after the eleventh passage (P11), and the consumptions of these three substances all increased at the twelfth passage (P12) and the thirteenth passage (P13) as well (FIG. 16).

From the results above, when the cell passage number is greater than or equal to a particular number, the TCA cycle becomes predominant over the glycolytic pathway to, for example, secure energy in a living body and thus a larger amount of pyruvic acid is consumed, resulting in the generation of a large amount of reactive oxygen species. It is understood that the synthesis of a large amount of glutathione, which is an antioxidative substance, is needed to remove such a large amount of reactive oxygen species generated, resulting in consumptions of a large amount of cystine and serine, which are necessary for the synthesis of glutathione. It is thus understood that the quality of mesenchymal stem cells can be evaluated using, as indicators, the consumptions of pyruvic acid, cystine, and serine in the medium by the cells. Furthermore, it is understood that changes in the consumptions of these three substances vary depending on the medium in which the cells are cultured, and a medium for mesenchymal stem cells can also be evaluated using the consumptions of these substances as indicators.

In addition, the results of the determination of whether the passage number is appropriate, obtained based on the consumptions of pyruvic acid, cystine, and serine above (after the seventh passage (P7) when using CiMS, and after the eleventh passage (P11) when using Cellartis), significantly corresponded to the results of the cell transplantation test using mice in Example 5. Therefore, from the viewpoint of the results of the animal experiment as well, it is understood that the consumptions of pyruvic acid, cystine, and serine by the cells used as indicators in the present invention are excellent indicators for evaluation of the quality of MSCs.

### Industrial Applicability

The present invention makes it possible to more reliably, more simply, and more quickly evaluate the quality of mesenchymal stem cells, which is thus useful. Using this evaluation method makes it possible to produce mesenchymal stem cells with stable quality (differentiation potential, proliferative capacity, undifferentiation properties, etc.), which is thus useful. Differentiating mesenchymal stem cells with stable quality also makes it possible to produce mesenchymal cells with stable quality more efficiently and reproducibly, which is thus useful. Furthermore, the mesenchymal stem cells and the mesenchymal cells obtained using the production method of the present invention have stable quality and are reproducibly produced, and are thus particularly suitable for application to the cell transplantation treatment and are useful. In addition, with the present invention, it is also possible to evaluate a medium for mesenchymal stem cells. Using this evaluation method makes it possible to select a medium with which deterioration of the quality of mesenchymal stem cells caused by passaging can be further avoided, thus making it possible to provide mesenchymal stem cells and mesenchymal cells with stable quality more efficiently and reproducibly, and the method is thus useful.

The present application claims the benefit of priority based on Japanese Patent Application No. 2022-059920 and Japanese Patent Application No. 2022-196791 filed in Japan, which are incorporated herein by reference in their entirety.

## Claims

1. A method for evaluating quality of mesenchymal stem cells, comprising a step of evaluating the cells based on
(i) measurements of consumptions of pyruvic acid, cystine, and serine by the cells, or
(ii) measurements of mitochondrial sizes in the cells.

2. The method according to claim 1, wherein the consumptions of pyruvic acid, cystine, and serine are calculated based on differences in abundances of pyruvic acid, cystine, and serine in a medium between before and after the start of culture of the cells.

3. The method according to claim 2, wherein the abundances are concentrations.

4. The method according to any one of claims 1 to 3, wherein the step of evaluating the cells is conducted based on differences in the consumptions of pyruvic acid, cystine, and serine between before and after a passage.

5. The method according to claim 4, further comprising a step of selecting cells in which the differences in the consumptions of pyruvic acid, cystine, and serine between before and after a passage are greater than or equal to predetermined values.

6. The method according to claim 4, further comprising a step of selecting cells in which the differences in the consumptions of pyruvic acid, cystine, and serine between before and after a passage are smaller than or equal to predetermined values.

7. The method according to claim 1, wherein the mitochondrial sizes are calculated using fluorescence intensities of fluorescence-labeled mitochondria.

8. The method according to claim 7, wherein the fluorescence intensities of the mitochondria reflect a mitochondrial charge.

9. The method according to any one of claims 1, 7, and 8, further comprising a step of selecting cells with a CV value smaller than or equal to a predetermined value.

10. A method for producing mesenchymal stem cells evaluated for quality, comprising a step of evaluating the cells based on
(i) measurements of consumptions of pyruvic acid, cystine, and serine by the cells, or
(ii) measurements of mitochondrial sizes in the cells.

11. Mesenchymal stem cells obtained using the method according to claim 10.

12. A method for producing mesenchymal cells, comprising a step of inducing differentiation of the mesenchymal stem cells according to claim 11.

13. Mesenchymal cells obtained using the method according to claim 12.

14. A cell transplantation therapy agent comprising the mesenchymal stem cells according to claim 11 or the mesenchymal cells according to claim 13.

15. A method for evaluating a medium for mesenchymal stem cells, comprising a step of evaluating the medium based on
(i) measurements of consumptions of pyruvic acid, cystine, and serine by the cells, or
(ii) measurements of mitochondrial sizes in the cells.

16. The method according to claim 15, wherein the consumptions of pyruvic acid, cystine, and serine are calculated based on differences in abundances of pyruvic acid, cystine, and serine in a medium between before and after the start of culture of the cells.

17. The method according to claim 15, wherein the mitochondrial sizes are calculated using fluorescence intensities of fluorescence-labeled mitochondria.
